(19) Europäisches Patentamt
European Patent Office
Office européen des brevets

(11) **EP 4 516 295 A1**

(12) **EUROPEAN PATENT APPLICATION**

(43) Date of publication:
**05.03.2025 Bulletin 2025/10**

(21) Application number: **23195151.8**

(22) Date of filing: **04.09.2023**

(51) International Patent Classification (IPC):
**A61K 9/51** (2006.01)

(52) Cooperative Patent Classification (CPC):
**A61K 9/5123; A61K 9/5192;** A61K 47/26

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **ETH Zurich**
**8092 Zurich (CH)**

(72) Inventors:
• **LEROUX, Jean-Christophe**
  **8053 Zürich (CH)**
• **MONTANARI, Elita**
  **8037 Zürich (CH)**
• **BADER, Johannes**
  **8032 Zürich (CH)**

(54) **PH-INDUCIBLE STRUCTURE-SWITCHING LIPID NANOVECTORS, SEMI-SYNTHETIC EXTRACELLULAR VESICLES, METHODS OF MAKING SAME AND USES THEREOF**

(57) The present disclosure provides a pH-inducible structure-switching non-lamellar lipid nanovector (LNV) comprising: (a) at least one ionizable cationic lipid; (b) at least one phospholipid displaying a critical packing parameter (CPP) value > 1; and (c) at least one non-ionic surfactant displaying a CPP value < 1, at a molar concentration of between 10 % and 50 %, a method of making the LNV, a semi-synthetic extracellular vesicle (ssEV) resulting from the fusion of the LNV with extracellular vesicles, a kit and a use of the ssEV as a medicament or diagnostic agent.

FIG. 1A

EP 4 516 295 A1

**Description**

FIELD OF THE DISCLOSURE

The present disclosure relates to pH-inducible structure-switching lipid nanovectors, semi-synthetic extracellular vesicle, methods of making same and uses thereof. More specifically, the present disclosure is concerned with lipid nanovectors having the ability to spontaneously fuse with extracellular vesicles.

BACKGROUND OF THE DISCLOSURE

Extracellular vesicles (EVs) are cell-derived vesicular structures and important intercellular communicators that transport macromolecules, such as messenger ribonucleic acids (mRNA) or proteins, through the extracellular space (O'Brien et al., 2020). Encapsulating large quantities of therapeutic nucleic acids such as short interfering RNA (siRNA) and mRNA into EVs remains a challenging task, and a versatile approach to achieving this task is still missing (De Jong et al., 2019). Active loading strategies generally involve disturbing the vesicle bilayer to allow drug encapsulation (De Jong et al., 2019). This approach is especially relevant for the loading of EVs with relatively high molecular weight drugs. For example, for nucleic acid-based therapeutics, electroporation has emerged as a popular approach (Alvarez-Erviti, et al., 2011). However, how destructive this method is and whether the biological cargo is lost during the loading process is not fully understood. Similarly, sonication has been used as a way to temporarily destabilize the membrane and load EVs with biologicals (Lamichhane et al., 2016).

Simpler, passive methods of loading macromolecules (*e.g.,* therapeutic macromolecules) in EVs that do not compromise EV functions are needed.

The present description refers to a number of documents, the content of which is herein incorporated by reference in their entirety.

SUMMARY OF THE DISCLOSURE

The present disclosure provides a lipid nanostructure (i.e., lipid nanovector (LNV)) able to spontaneously hybridize/fuse with EVs. This passive process does not need further external stimuli (*i.e.,* sonication, electroporation, specific buffer components) and occurs at very mild conditions (*e.g.,* physiological conditions at pH 7.4, 37°C) within minutes. The LNVs promote fusion stalk in EVs leading to semi-synthetic EV particles (ssEVs) without compromising the EVs biological activity. These versatile particles may be loaded with various molecular and/or macromolecular therapeutics.

The present disclosure also provides a method of making the LNVs and ssEVs.

Without being so limited, it is hypothesized that the intrinsic fusogenic property of the LNVs of the present disclosure is based on their non-lamellar lyotropic liquid crystal (LLC)-based structure (*e.g.,* sponge-based structure).

More specifically, in accordance with the present disclosure, there are provided the following items:

Item 1. A pH-inducible structure-switching non-lamellar lipid nanovector (LNV) comprising:

(a) at least one ionizable cationic lipid;

(b) at least one phospholipid displaying a critical packing parameter (CPP) value > 1; and

(c) at least one non-ionic surfactant displaying a CPP value < 1, at a molar concentration of between 10 % and 50 %.

Item 2. The LNV of item 1, wherein:

(a) the at least one ionizable cationic lipid has an apparent acid dissociation constant (pKa) prior to incorporation in LNV of between 5 and 7.5;

(b) the at least one phospholipid is a phosphatidylethanolamine (PE)-based lipid;

(c) the at least one surfactant is a polyethylene glycol ester of fatty acid or a polysorbate; or

(d) a combination of at least two of (a) to (c).

Item 3. The LNV of item 1 or 2, wherein:

(a') the at least one ionizable cationic lipid is D-Lin-MC3-DMA (MC3);

(b') the at least one phospholipid is 2-dioleoyl-sn-glycero-3-phosphoethanolamine (DOPE);

(c') the at least one non-ionic surfactant is a polyethylene glycol ester of fatty acid, such as polyethylene glycol 12-hydoxystearate, preferably Kolliphor® HS15 (KLP); or

(d') a combination of at least two of (a') to (c').

Item 4. The LNV of any one of items 1 to 3, loaded with a drug or a diagnostic agent, preferably wherein the drug or diagnostic agent is a nucleic acid molecule.

Item 5. Composition comprising the LNV defined in any one of items 1 to 4, and at least one pharmaceutically acceptable excipient.

Item 6. Method for making the LNV defined in any one of items 1 to 3, comprising mixing (a) an organic phase comprising the at least one ionizable cationic lipid, the at least one phospholipid and the at least one surfactant, in an organic solvent; and (b) an aqueous medium.

Item 7. The method of item 6, wherein at least one of (i) the organic phase further comprises a lipophilic drug or diagnostic agent; and (ii) the aqueous phase further comprises a hydrophilic a drug or diagnostic agent, preferably wherein the hydrophilic drug or diagnostic agent is a nucleic acid molecule.

Item 8. The method of item 7, wherein (ii) the aqueous phase further comprises a hydrophilic drug or diagnostic agent and the aqueous medium has a pH of between about 4 and about 6.

Item 9. The method of any one of items 6 to 8, wherein the mixing is performed with a microfluidic device.

Item 10. Method of making a semi-synthetic extracellular vesicle (ssEV) comprising mixing the LNV defined in any one of items 1 to 4 or the composition defined in item 5, with an extracellular vesicle (EV) at a pH higher than 6 and up to about 10, whereby the ssEV is generated.

Item 11. Semi-synthetic extracellular vesicle (ssEV) prepared by the method defined in item 10.

Item 12. Semi-synthetic extracellular vesicle (ssEV) comprising essentially all components, partially or completely mixed, of:

(a) the LNV defined in any one of items 1 to 4; and

(b) an extracellular vesicle (EV).

Item 13. Composition comprising the ssEV defined in item 11 or 12, and at least one pharmaceutically acceptable excipient.

Item 14. Kit comprising (A) (a) the LNV defined in any one of items 1 to 4 or the composition defined in item 5; and (b) (i) a solution for hydrating (a); (ii) extracellular vesicles (EVs) or a composition comprising the EVs and at least one pharmaceutically acceptable excipient; (iii) instructions for using (a) and at least one of (i) and (ii); or (iv) a combination of at least two of (i) to (iii); or (B) (a') the ssEV defined in item 11 or 12, or the composition defined in item 13; and (b') (i') a solution for hydrating (a'); (ii') instructions for using (a') and (i'); or (iii') a combination of (i') and (ii').

Item 15. Use of the ssEV defined in item 11 or 12 or of the composition defined in item 13, as a medicament or a diagnostic agent.

Other objects, advantages and features of the present disclosure will become more apparent upon reading of the following non-restrictive description of specific embodiments thereof, given by way of example only with reference to the accompanying drawings.

BRIEF DESCRIPTION OF THE DRAWINGS

In the appended drawings:

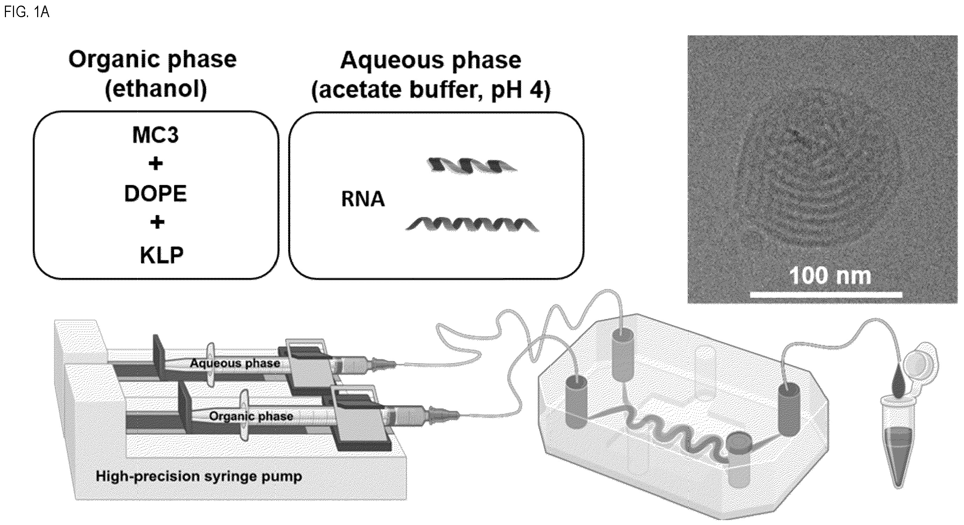

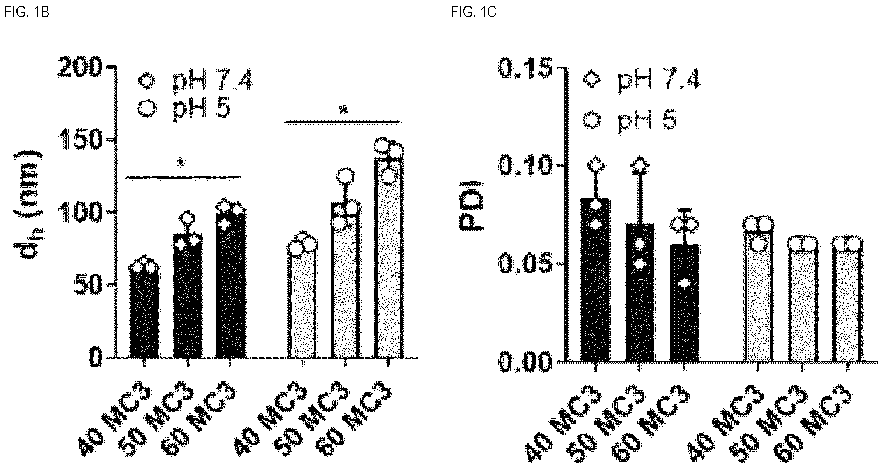

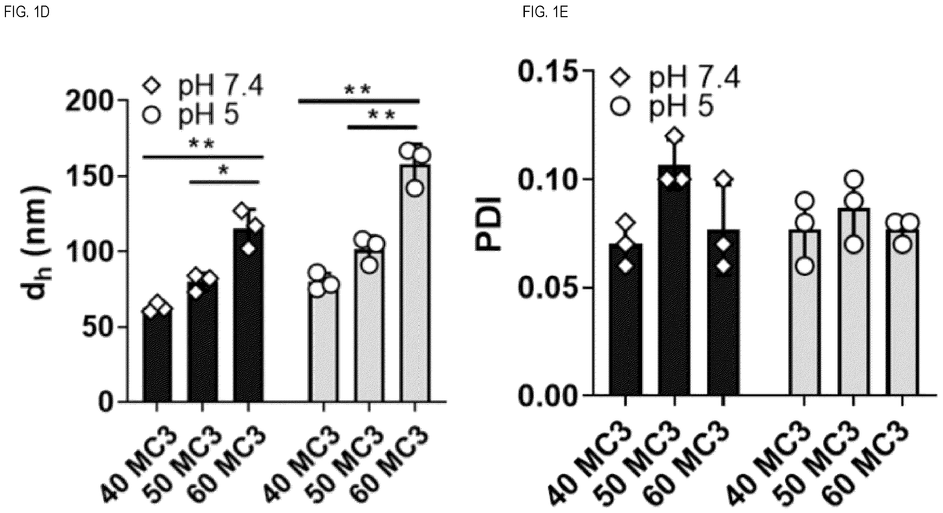

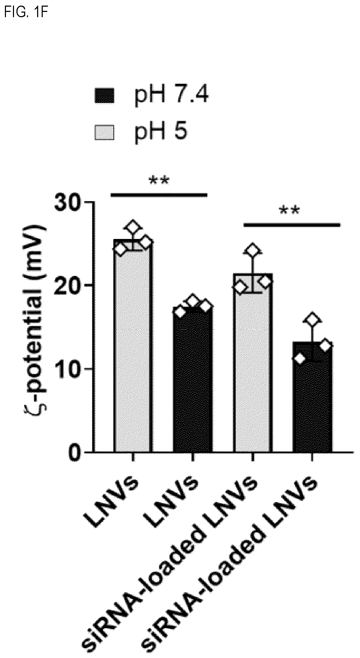

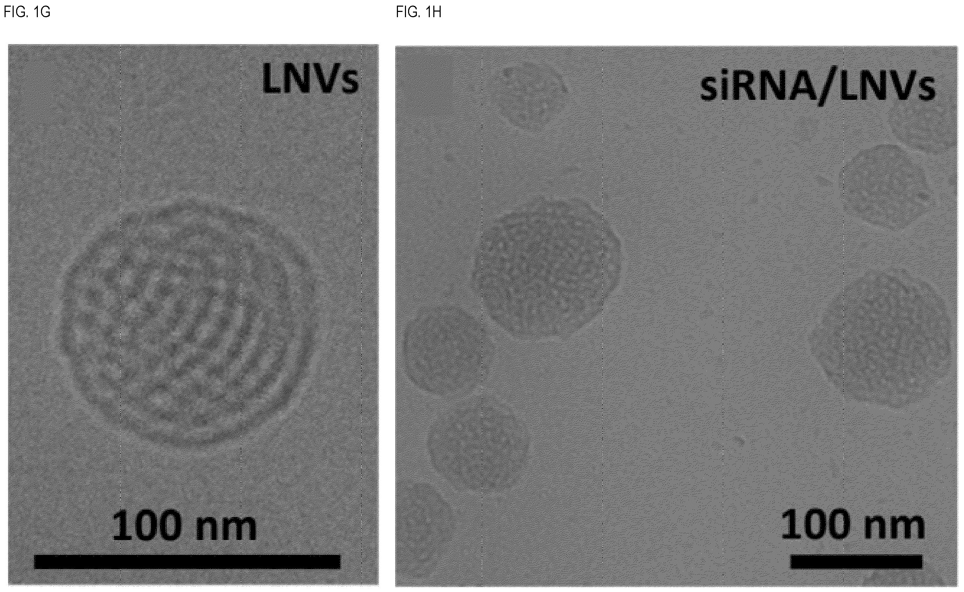

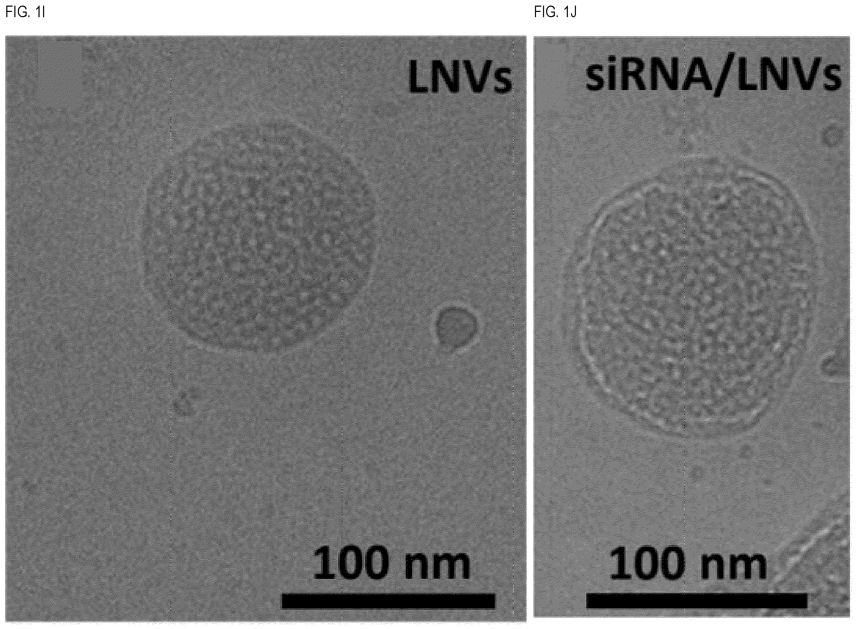

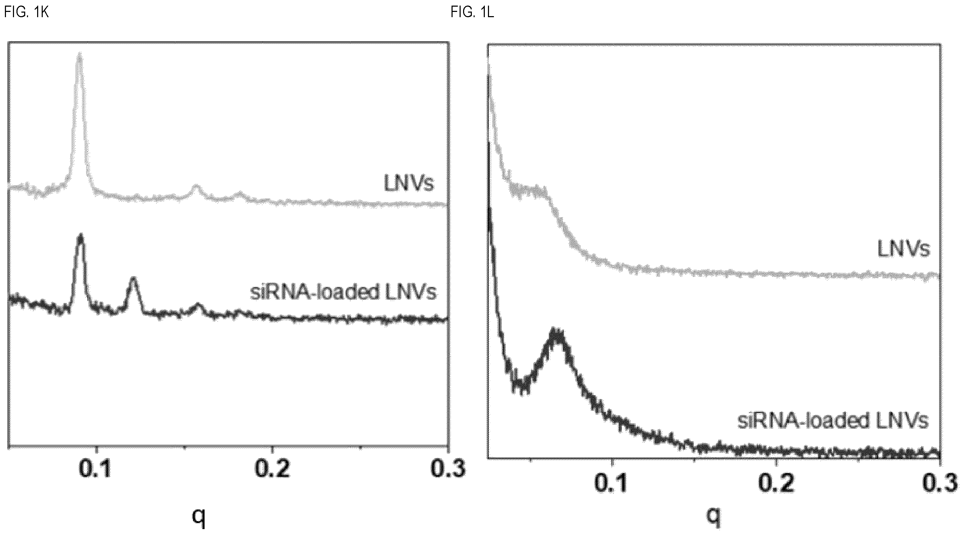

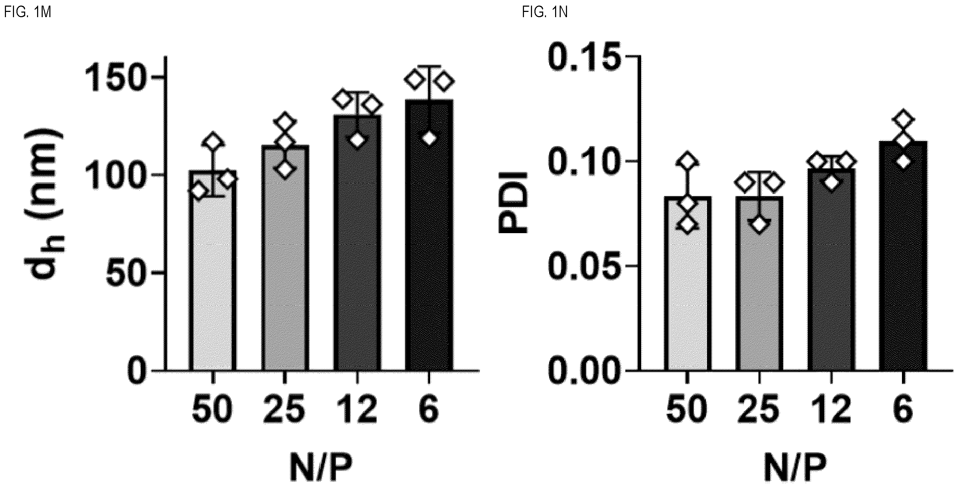

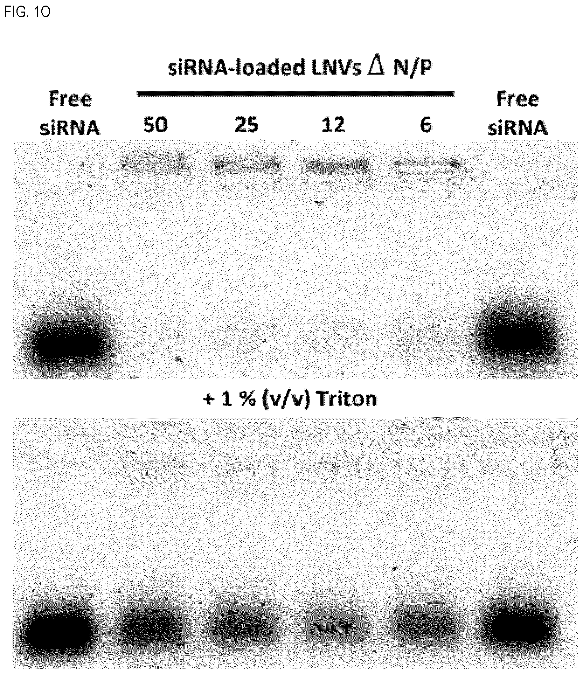

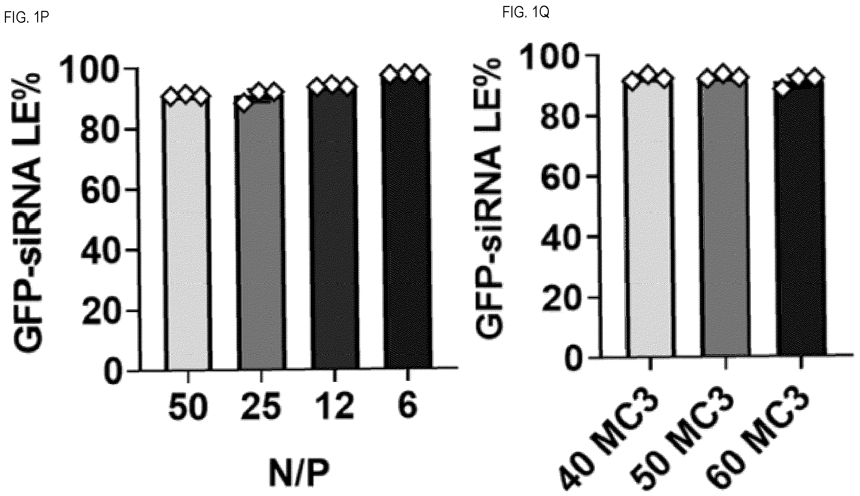

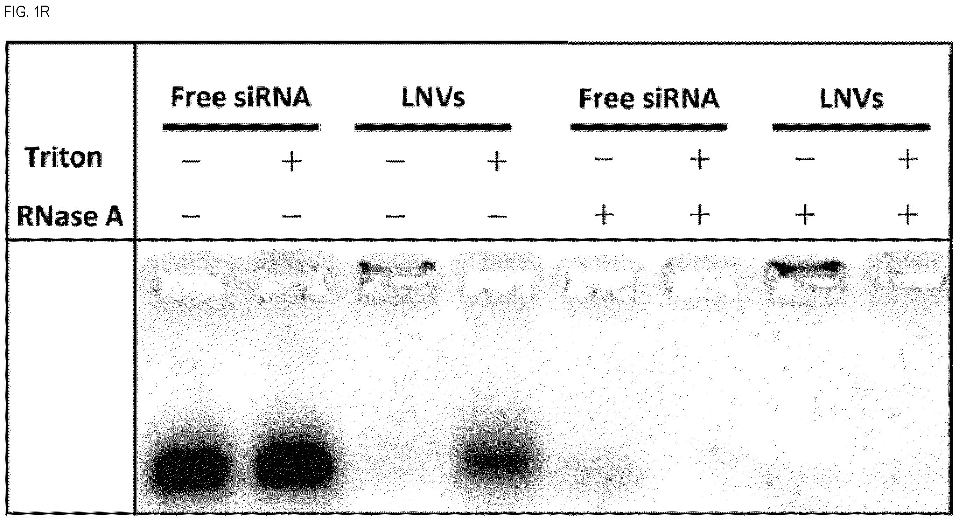

FIGs. 1A-R: Composition, preparation process and physicochemical characterization of unloaded vs siRNA-loaded LNVs produced with a microfluidic device with varying molar concentrations of MC3 (40, 50 and 60 mol %) and N/P ratios (ratio of positively-chargeable lipid amine (N = nitrogen) groups to negatively-charged nucleic acid phosphate (P) groups). (FIG. 1A) show materials and procedure with which siRNA-loaded LNVs used in Examples herein are produced. Created with BioRender.com. (FIG. 1B) and (FIG. 1C) represent the hydrodynamic diameter ($d_h$) and polydispersity index (PDI), respectively of unloaded LNVs; while (FIG. 1D) and (FIG. 1E) represent the $d_h$ and PDI, respectively of siRNA-loaded LNVs, at pH 7.4 and 5 prepared with different lipid ratios (40, 50 and 60 mol % MC3, N/P 25). (FIG. 1F) shows $\zeta$-potential ($\zeta$-pot) values of LNVs vs. siRNA-loaded LNVs at pH 7.4 and 5 (N/P = 25, 60 mol % MC3). (FIG. 1G) and (FIG. 1H) display cryo-transmission electron microscopy (cryo-TEM) images of LNVs and siRNA-loaded LNVs (60 % MC3), respectively at pH 7.4, whereas (FIG. 1I) and (FIG. 1J) display corresponding images at pH 5. (FIG. 1K) and (FIG. 1L) show small-angle X-ray scattering (SAXS) analysis of LNVs vs. siRNA-loaded LNVs (N/P = 25, 60 mol % MC3) at pH 5 and 7.4, respectively. (FIG. 1M) and (FIG. 1N) show size and PDI, respectively, of siRNA-loaded LNVs with several N/P ratios (*i.e.,* 6, 12, 25 and 50) and 60 mol % MC3, at pH 7.4. (FIG. 1O) displays electrophoretic mobility shift assay (EMSAs) of siRNA-loaded LNVs at different N/P ratios (*i.e.,* 6, 12, 25 and 50) and 60 mol % MC3 with and without Triton™ X-100. (FIG. 1P) and (FIG. 1Q) show the loading efficiency (LE %) of siRNA in LNVs at different N/P ratios (*i.e.,* 6, 12, 25 and 50) and 60 mol % MC3, and material ratios (40, 50 and 60 mol% MC3), as assessed with the RiboGreen™ assay. (FIG. 1R) shows the integrity of siRNA loaded into LNVs (N/P = 25, 60 mol % MC3) with EMSA, after RNase treatment at 37 °C. Statistical significance was evaluated by Two-way Anova, Tukey's multiple comparison Test (**$P \leq 0.01$ and *$P \leq 0.05$). All the experiments are performed in triplicate (n = 3).

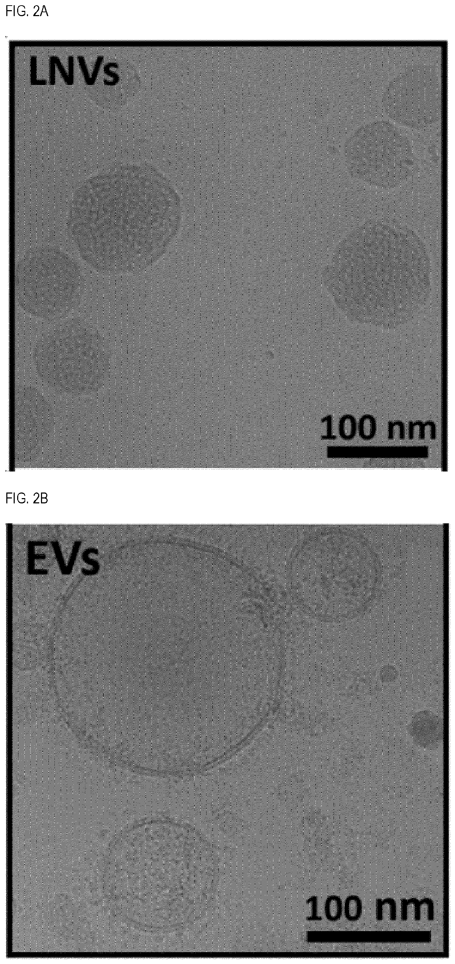

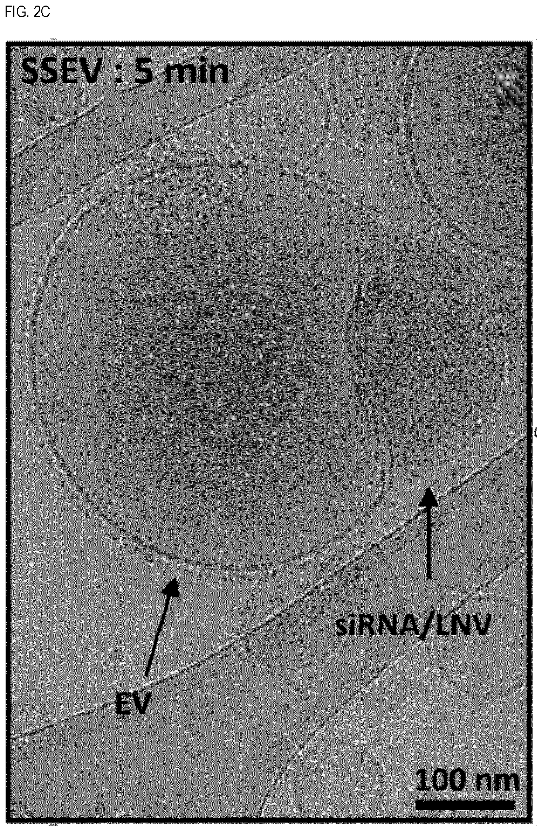

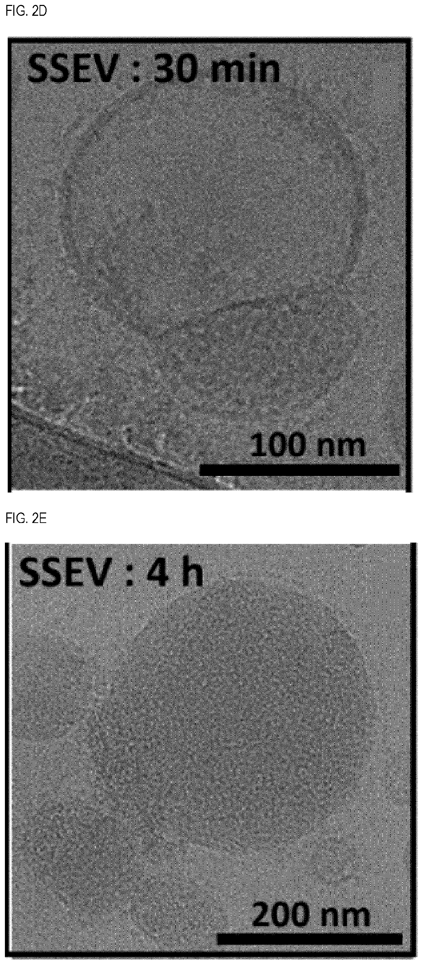

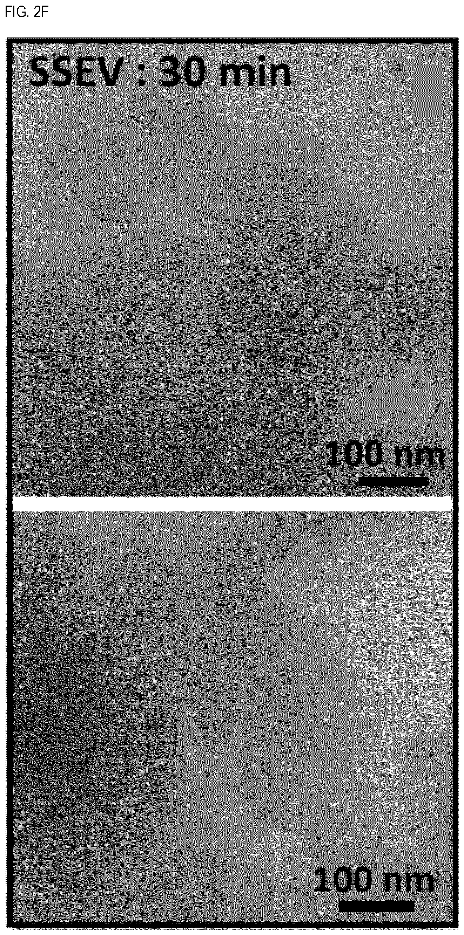

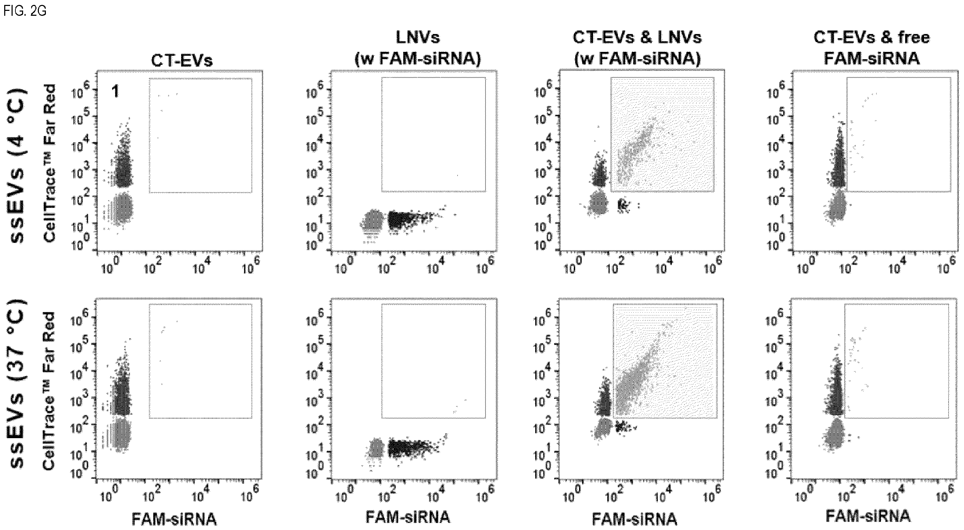

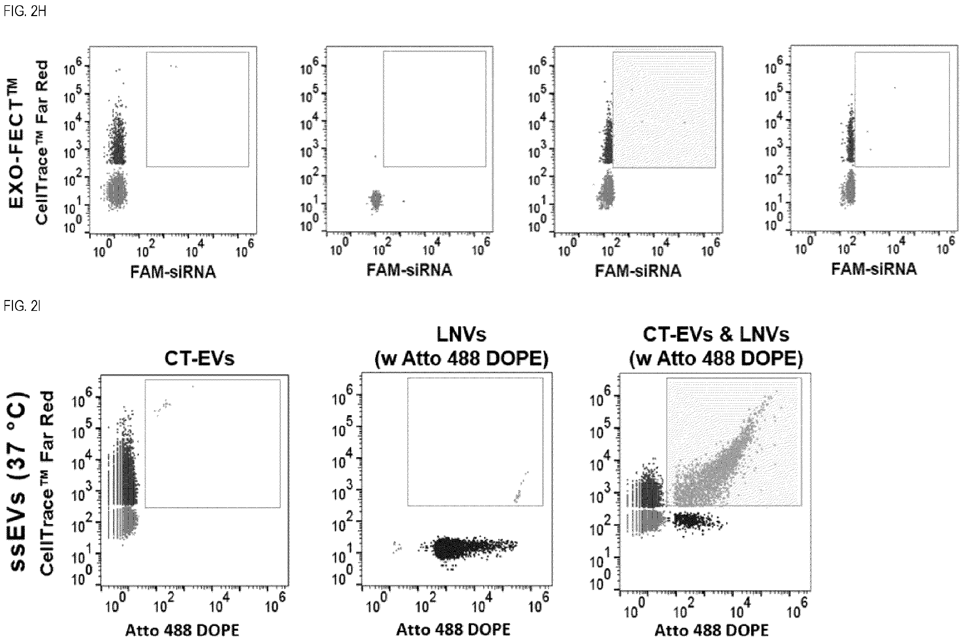

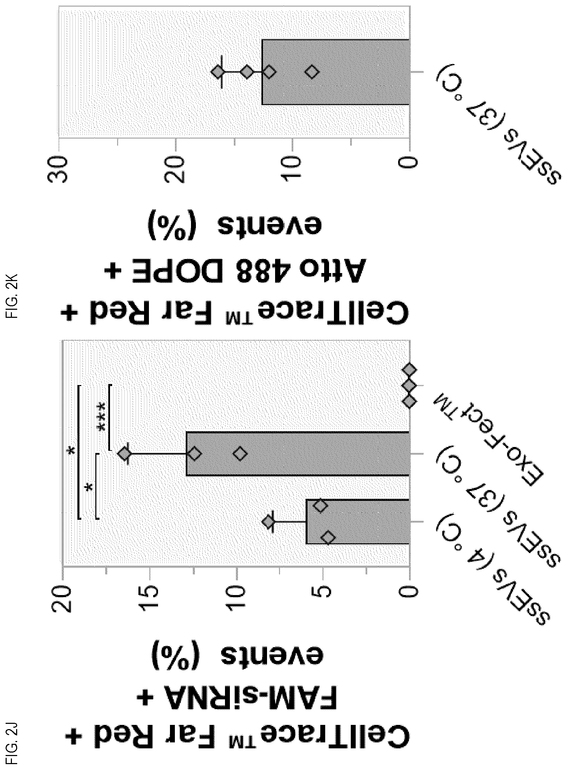

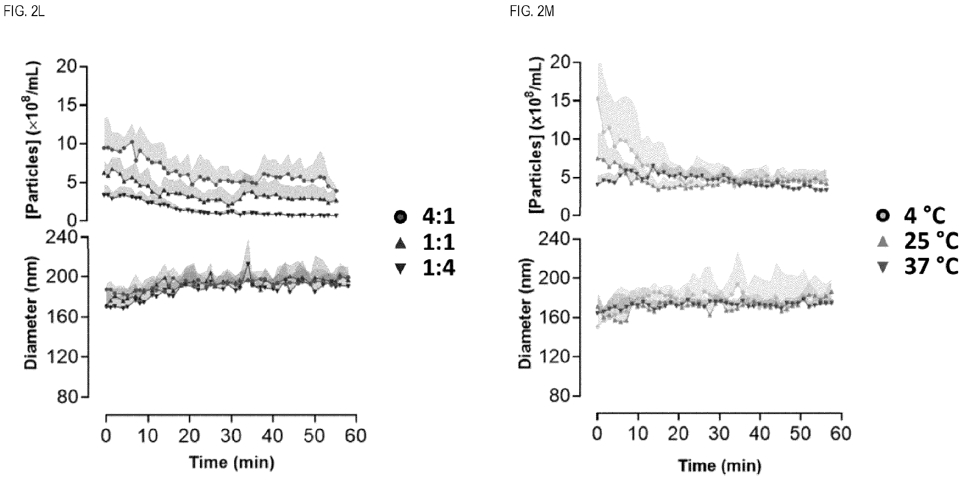

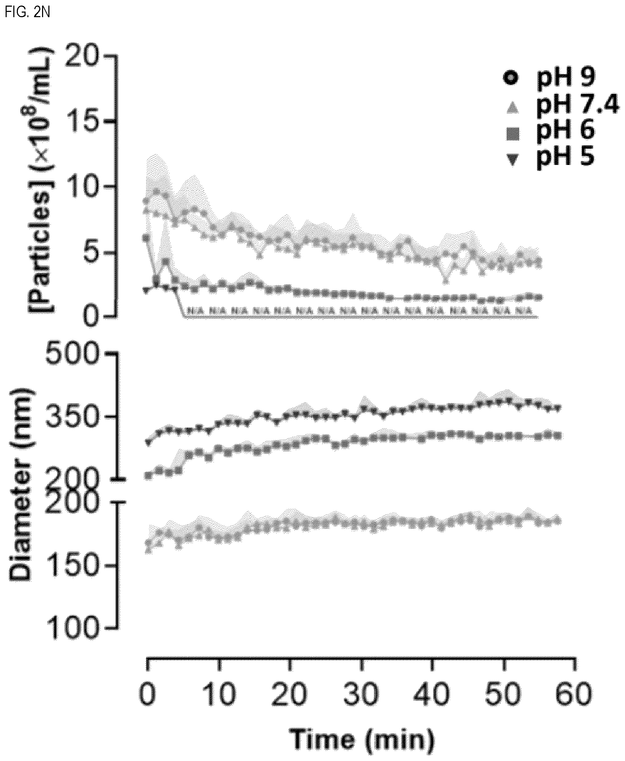

FIGs. 2A-N: Cryo-TEM images of siRNA-loaded LNVs (60 mol % MC3, N/P 25) (FIG. 2A), MSC-derived EVs (FIG. 2B) and ssEVs after 5 minutes (FIG. 2C), 30 minutes (FIG. 2D) and 4 hours (FIG. 2E) at 37 °C and pH 7.4. (FIG. 2F) shows the cryo-TEM images of siRNA-loaded LNVs (60 mol % MC3, N/P 25) and MSC-derived EVs mixture at pH 5 after 30 minutes. The data suggests that a pH of 6 or lower does not lead to ssEV formation; instead, a massive aggregation is observed. (FIGs. 2G-I) show bivariate dot plots of CellTrace™ Far Red vs. FAM-siRNA in LNVs and with the Exo-Fect™ transfection reagent (FIGs. 2G-H) and with Atto 488-labeled DOPE (FIG. 2I). (FIG. 2J) Loading of FAM-siRNA (as shown in percentage of the double positive population CellTrace™ Far Red over FAM-siRNA) into ssEVs by using LNVs (N/P 25 and 60 mol % MC3) or the commercially available Exo-Fect™ Exosome Transfection Kit. (FIG. 2K) Transfer of Atto 488 DOPE (as shown in percentage of the double positive population CellTrace™ Far Red over Atto 488 DOPE) from LNVs to ssEVs. (FIGs. 2L-N) Dynamic and static light scattering (DLS/SLS) plate reader analysis of the ssEV formation at different LNV/EV number ratios (FIG. 2L), temperatures (FIG. 2M) and pH values (FIG. 2N) over 1 h. Statistical significance was evaluated by One-way Anova, Tukey post hoc test (****$P \leq 0.0001$, ***$P \leq 0.001$, **$P \leq 0.01$ and *$P \leq 0.05$). Experiments are performed in triplicate (n = 3).

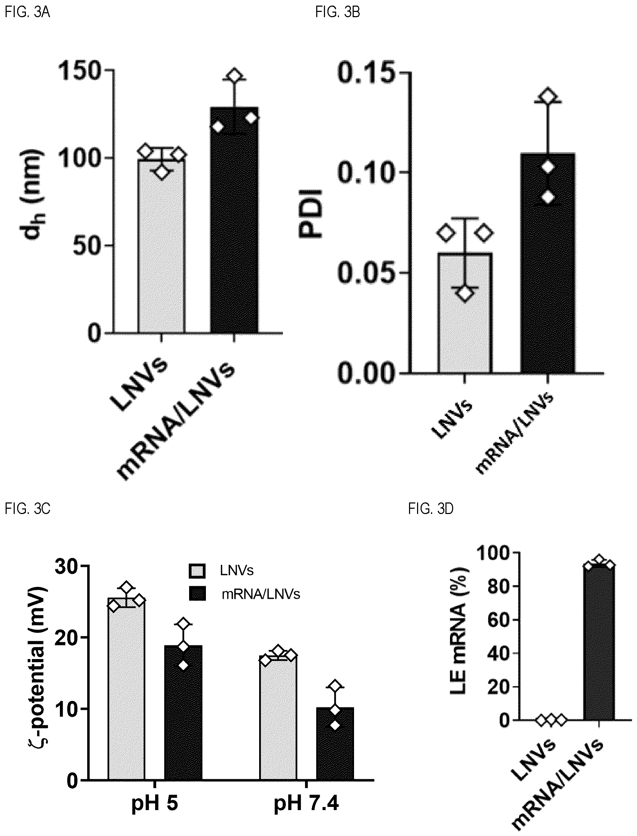

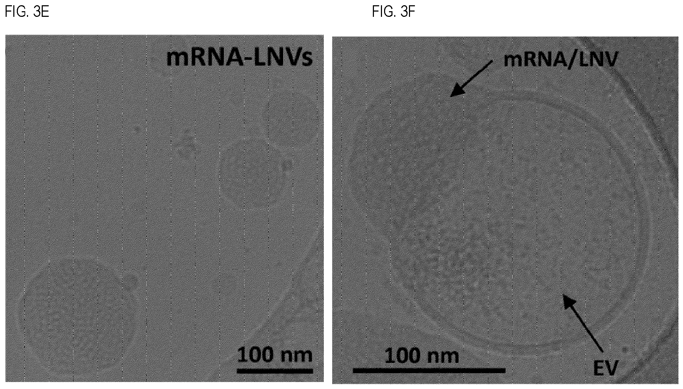

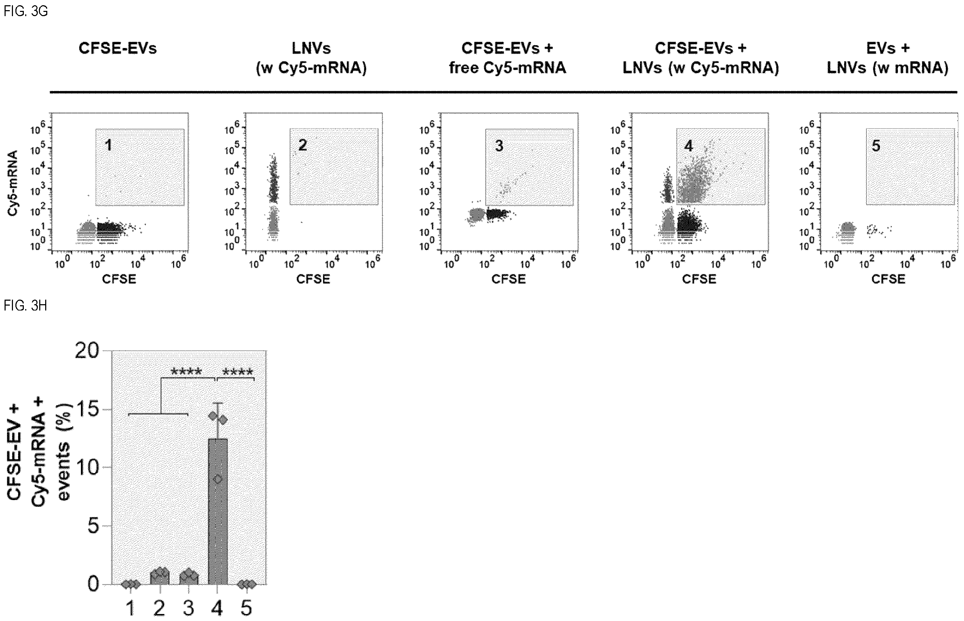

FIGs. 3A-H: $d_h$ (FIG. 3A), and PDI (FIG. 3B) of LNVs vs. mRNA-loaded LNVs at pH 7.4, N/P 0 (unloaded LNVs) and 25 and 60 mol % MC3 as measured by DLS. $\zeta$-pot (FIG. 3C) of LNVs vs. mRNA-loaded LNVs at pH 5 and 7.4, with N/P 0 (unloaded LNVs) and 25 and 60 mol % MC3. (FIG. 3D) displays LE % of mRNA in LNVs analyzed with the RiboGreen assay (N/P 25 and 60 mol % MC3). All the experiments are performed in triplicate (n = 3). (FIGs. 3E-F) show cryo-TEM images of mRNA-loaded LNVs (FIG. 3E), and the ability of mRNA-loaded LNVs to fuse with EVs and form ssEVs (FIG. 3F) at pH 7.4, 37 °C, incubation time of 5 min N/P 25 and 60 mol % MC3. (FIGs. 3G-H) show bivariate dot plots of CFSE-EVs alone, Cy5-mRNA-loaded LNVs alone, CFSE-EVs with free Cy5-mRNA, CFSE-EVs with Cy5-mRNA-loaded LNVs and EVs with mRNA-loaded LNVs. The double positive population (double positive population in upper right square insert) appears upon mixing CFSE-EVs with Cy5-mRNA-loaded LNVs and confirms the formation of ssEVs. CFSE: carboxyfluorescein succinimidyl ester. Mean $\pm$ s.d. (n = 3). Statistical significance was evaluated by One-way Anova, Tukey post hoc test (****$P \leq 0.0001$, ***$P \leq 0.001$, **$P \leq 0.01$ and *$P \leq 0.05$). Experiments are performed in triplicate (n = 3).

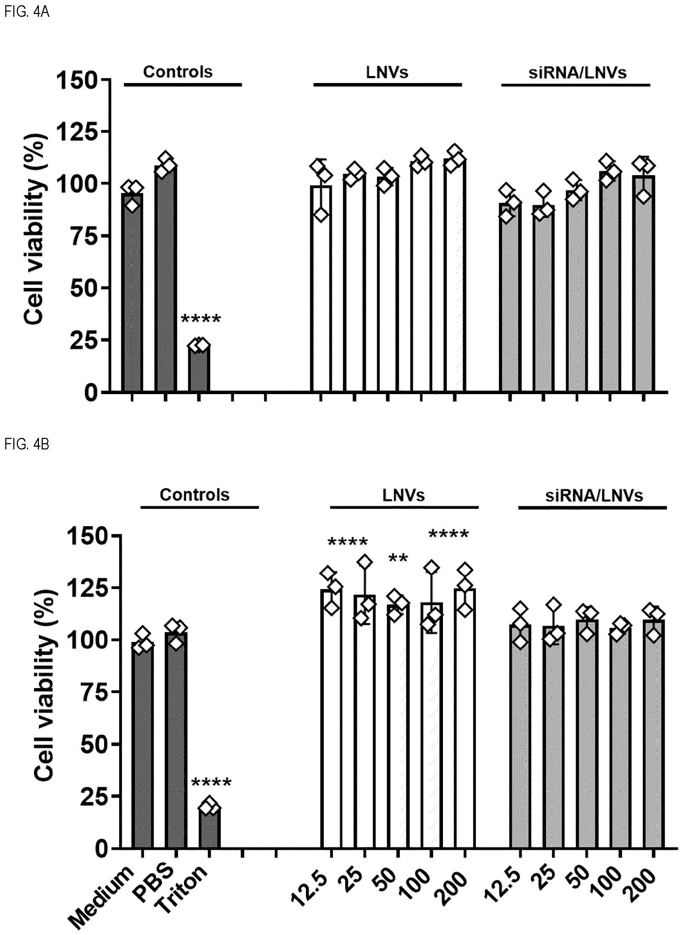

FIGs. 4A-B: Cell viability of HeLa cells treated with LNVs and siRNA-loaded LNVs (siRNA/LNVs) after 4 h (FIG. 4A) and 24 h (FIG. 4B) incubation, determined with a MTS assay (colorimetric method for the sensitive quantification of viable cells) (60 mol % MC3; N/P 25), at lipid concentrations ranging from 12.5 to 200 $\mu$g/mL. Mean $\pm$ s.d. (n = 3). All samples are compared to the negative control (medium). Statistical significance was evaluated by One-way Anova, Tukey post hoc Test (*$P \leq 0.05$, **$P \leq 0.01$ and ****$P \leq 0.0001$).

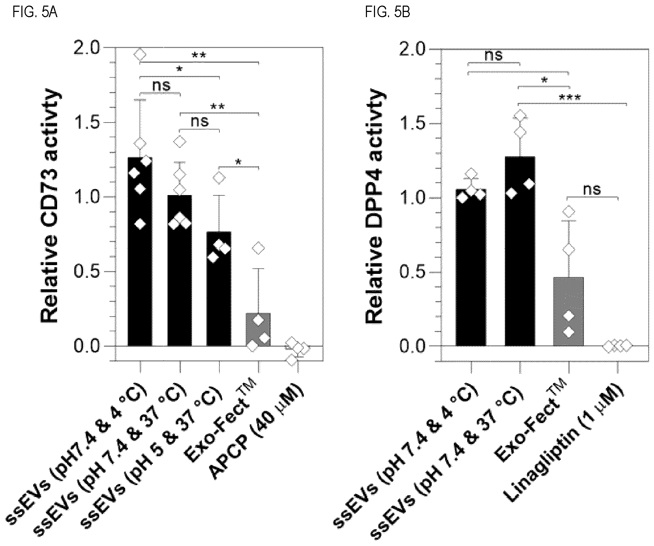

FIGs. 5A-B: Enzymatic activity of two EV-specific membrane proteins CD73 (FIG. 5A) and DPP4 (FIG. 5B) isolated from the bone marrow-derived mesenchymal stem cell (MSC) line HS-5. CD73 and DPP4 activity was determined with an absorbance and luminescence-based assay, respectively, after ssEV formation (incubation for 30 min in HEPES-buffered saline (HEBS), at 4 °C and 37 °C, pH 7.4, or at 37 °C, pH 5 for 30 min) and compared to that obtained with Exo-Fect™ (incubation at 37 °C for 10 min in HEBS buffer pH 7.4). Adenosine 5'-($\alpha,\beta$-methylene)diphosphate (APCP, 40 $\mu$M) in the case of CD73 and linagliptin (1 $\mu$M) in the case of DPP4 served as inhibitor controls. Results were normalized to the enzymatic activity of EVs that were subjected to identical fusion conditions in the absence of LNVs.

Mean $\pm$ s.d. (n = 4-6). Statistical significance was evaluated by unpaired, two-tailed Student's t-test with Welch's correction (***P $\leq$ 0.001, **P $\leq$ 0.01 and *P $\leq$ 0.05) (n = 4-6).

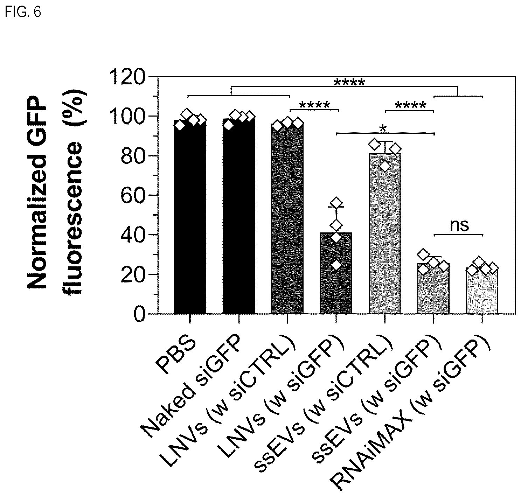

FIG. 6: Comparison of siRNA transfection in HeLa cells expressing the green fluorescent protein (GFP). GFP-siRNA-loaded LNVs (N/P 25 and 60 mol % MC3) (LNVs (w siGFP)) and LNVs loaded with a scrambled control siRNA (LNVs (w siCTRL)) (60 mol % MC3; N/P 25) are compared with GFP-siRNA-loaded ssEVs (ssEVs (w siGFP)), Control-siRNA-loaded ssEVs (ssEVs (w siCTRL)), Lipofectamine™ RNAiMAX with GFP-siRNA (RNAiMAX (w siGFP)), PBS, and naked GFP-siRNA (Naked siGFP). After 48 h in 10 % serum transfection efficiencies are assessed by measuring fluorescence signals with flow cytometry. 5 pmol of GFP-siRNA in each formulation was added per well (corresponding to a concentration of 10 nM). Mean $\pm$ s.d. (n = 3). Statistical significance was evaluated by One-way Anova, Tukey post hoc test (****P $\leq$ 0.0001, ***P $\leq$ 0.001, **P $\leq$ 0.01 and *P $\leq$ 0.05) (n = 3).

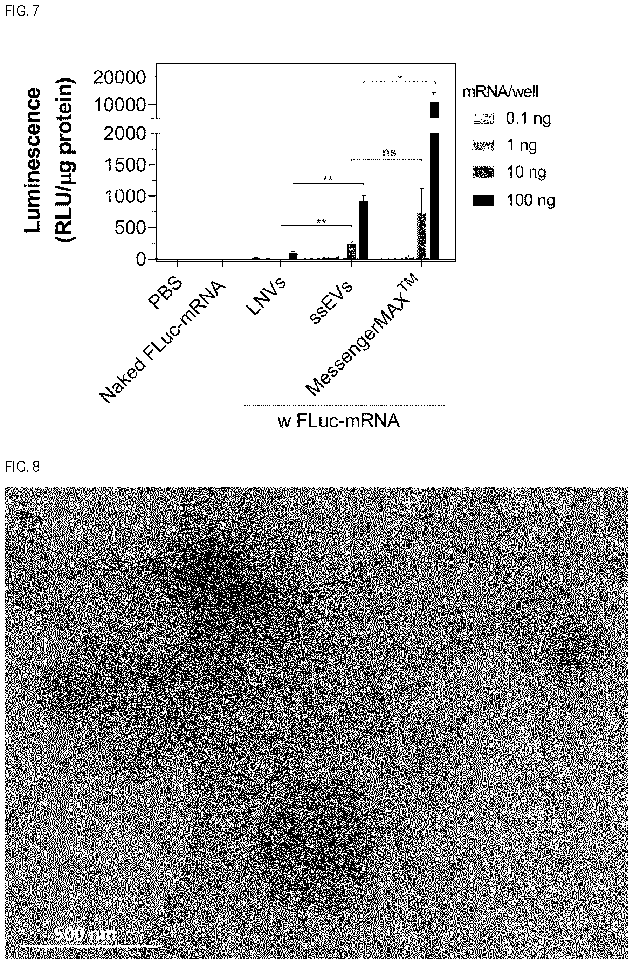

FIG. 7: Comparison of Firefly Luciferase (FLuc)-mRNA transfection in HeLa cells. FLuc-mRNA-loaded LNVs (LNVs) (N/P 25 and 60 mol % MC3) and FLuc-mRNA-loaded ssEVs (ssEVs) are compared to PBS, naked FLuc-mRNA and Lipofectamine™ MessengerMAX™ (MessengerMax™) with FLuc-mRNA (w FLuc-mRNA). After 24 h incubation in ~ 90 % serum, transfection efficiencies were analyzed by measuring the expression of luciferase in cell lysates. HeLa cells were treated with 4 different mRNA doses (100 ng, 10 ng, 1 ng and 0.1 ng per well). Mean $\pm$ s.d. Statistical significance was evaluated by unpaired, two-tailed Student's t-test with Welch's correction (**P $\leq$ 0.01 and *P $\leq$ 0.05) (n = 3).

FIG. 8: Representative cryo-TEM images of particles produced with DOPE and KLP (48:52 mol %, respectively), without MC3, using a microfluidic device.

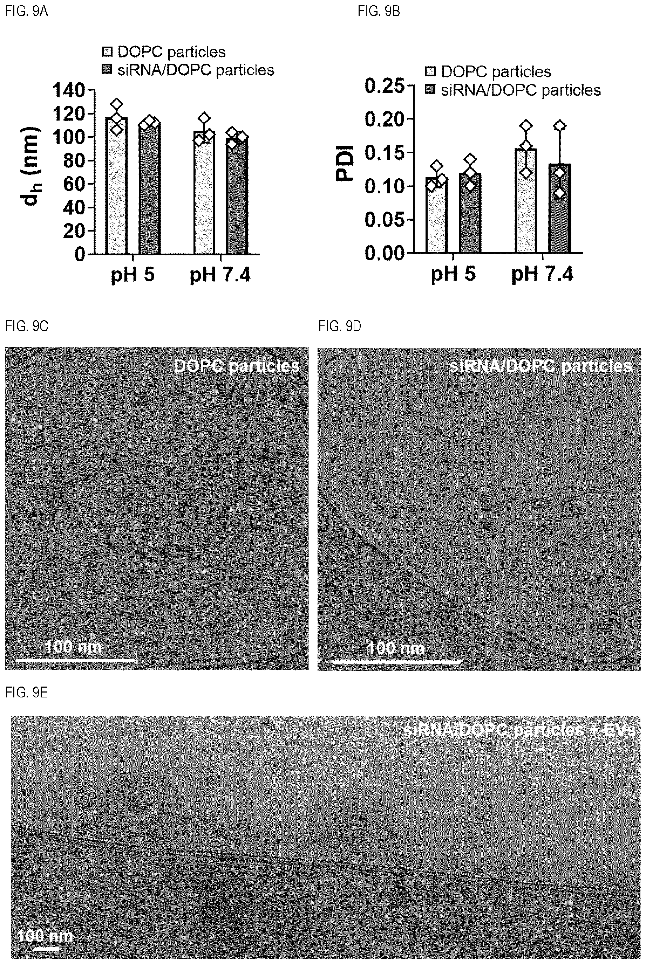

FIGs. 9A-E: Size ($d_h$), PDI and representative cryo-TEM images of particles and siRNA-loaded particles and their mixture with EVs that are produced with MC3, 1,2-dioleoyl-sn-glycero-3-phosphocholine (DOPC) and KLP (60:18:22 mol %, respectively, N/P 0 (unloaded LNVs) and 25), using a microfluidic device.

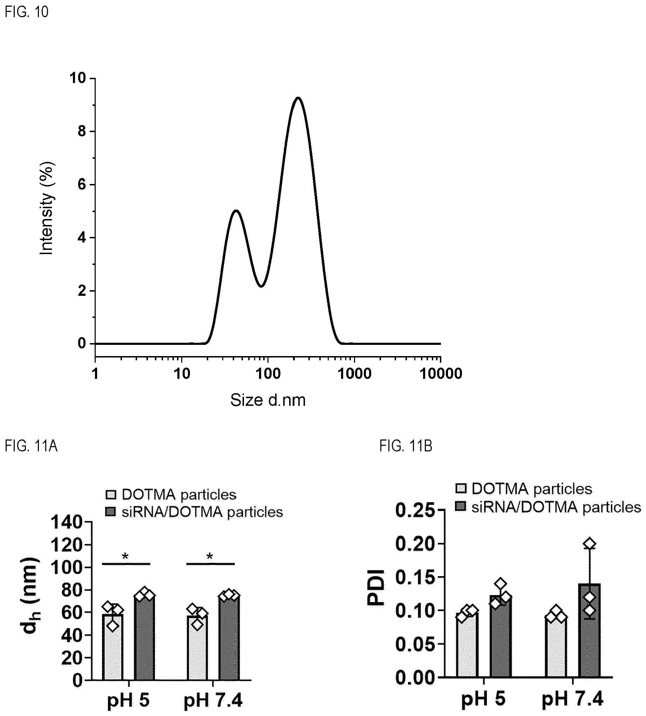

FIG. 10: Size distribution of nanoparticles that are produced with MC3, DOPE and 1-palmitoyl-2-hydroxy-sn-glycero-3-phosphocholine (MPPC) (60:18:22 mol %, respectively), using a microfluidic device.

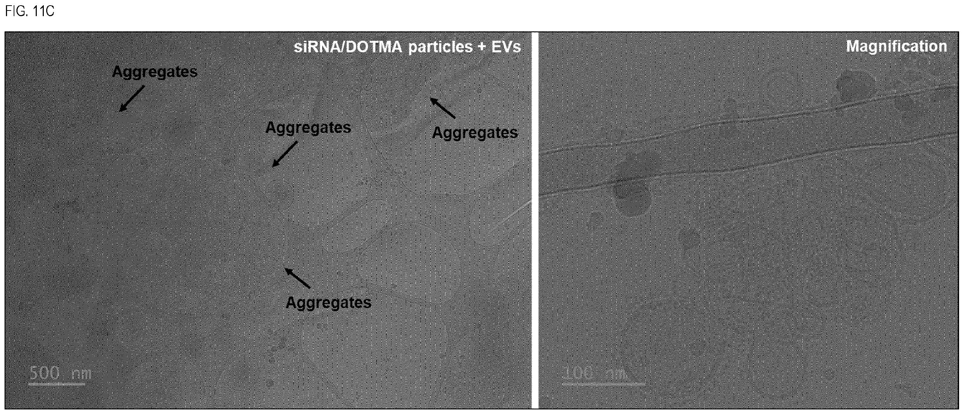

FIGs. 11A-C: Size ($d_h$) (FIG. 11A) and PDI (FIG. 11B) of unloaded nanoparticles and siRNA-loaded nanoparticles that are produced with the fixed cationic lipid 1,2-di-O-octadecenyl-3-trimethylammonium propane (DOTMA), DOPE and KLP (60:18:22 mol %, respectively, with N/P 0 and 25), at pH 5 and 7.4 using a microfluidic device. FIG. 11C shows representative cryo-TEM images of the mixture of siRNA-loaded particles (60 mol % DOTMA, N/P 25) and MSC-derived EVs.

## DESCRIPTION OF ILLUSTRATIVE EMBODIMENTS

### DEFINITIONS

*LNV components*

The present disclosure provides pH-inducible structure-switching non-lamellar LNVs. LNVs of the present disclosure are pH-inducible structure-switching in that they are able to transition from a hexagonal phase at an acidic pH (e.g., lower than about 6) to a sponge phase at a neutral pH *(e.g.,* about 6.5 or more) and vice-versa. The LNVs of the present disclosure comprise at least one ionizable cationic lipid, at least one phospholipid displaying a CPP > 1 and at least one non-ionic surfactant displaying a CPP value < 1, these components conferring to the LNVs their non-lamellar structure and their pH-inducible structure-switching properties. The sponge-based structure of LNVs enables a successful fusion with EVs, forming ssEVs displaying biological activity retention. The LNVs of the present disclosure are not vesicles.

The LNVs' average size ($d_h$) is between about 20 nm and about 200 nm.

Ionizable cationic lipids

The function of ionizable cationic lipids (*i.e.* lipids that switch their charge depending on the pH) in the LNVs of the present invention is 1) to complex a negatively charged drug (*e.g.*, nucleic acids such as RNAs) at acidic pH; 2) to produce a pH-inducible structure-switching non-lamellar LNV together with the phospholipid having a CPP > 1 (*e.g.*, 1,2-Dioleoyl-*sn*-glycero-3-phosphoethanolamine (DOPE)) and the non-ionic surfactant displaying a CPP value < 1 (*e.g.*, KLP); and 3) to support the fusion stalk process with EVs. In specific embodiments, ionizable cationic lipids of the present disclosure have an apparent acid dissociation constant (pKa) (prior to incorporation in LNVs) between 5 and 7.5 (or between 5.3 and 7.4; between 5.4 and 7.4; between 5.5 and 7.4; or between 5.6 and 7.4, or between 5.7 and 7.4, or between 5.8 and 7.4, or between 5.9 and 7.4, or between 6 and 7.4; or between 5.3 and 7.3; between 5.4 and 7.3; between 5.5 and 7.3; or between 5.6 and 7.3, or between 5.3 and 7.3, or between 5.8 and 7.3, or between 5.9 and 7.3, or between 6 and 7.3; or between 5.3 and 7.2; between 5.4 and 7.2; between 5.5 and 7.2; or between 5.6 and 7.2, or between 5.3 and 7.2, or between 5.8 and 7.2, or between 5.9 and 7.2, or between 6 and 7.2; or between 5.3 and 7.1; between 5.4 and 7.1; between 5.5 and 7.1; or between 5.6 and 7.1, or between 5.3 and 7.1, or between 5.8 and 7.1, or between 5.9 and 7.1, or between 6 and 7.1; or between 5.3 and 7; between 5.4 and 7; between 5.5 and 7; or between 5.6 and 7, or between 5.3 and 7, or between 5.8 and 7, or between 5.9 and 7, or between 6 and 7); and/or are dimethylamino (DMA) ionizable cationic lipid derivatives; and/or are cone-shaped ionizable cationic lipids. In specific embodiments, ionizable lipids of the present disclosure are (DMA) butanoate ionizable cationic lipid derivatives. Without being so limited, in specific embodiments, ionizable cationic lipids of the present disclosure are 4-(dimethylamino)-butanoic acid, (10Z,13Z)-1-(9Z,12Z)-9,12-octadecadien-1-yl-10,13-nonadecadien-1-yl ester also called DLin-MC3-DMA (MC3, pKa = 6.44), N,N-dimethyl-2-(2-((10Z,13Z)-octadeca-10,13-dien-1-yl)-2-((9Z,12Z)-octadeca-9,12-dien-1-yl)-1,3-dioxolan-4-yl)ethan-1-amine also called DLin-KC2-DMA (KC2, pKa 6.68), 1,2-dilinoleyloxy-N,N-dimethyl-3-aminopropane also called DLin-DMA (pKa = 6.8), 3-(dimethylamino)propane-1,2-diyl (9Z,9'Z,12Z,12'Z)-bis(octadeca-9,12-dienoate) also called DLin-DAP (pKa = 6.2), N,N-dimethyl-1-(2-((10Z,13Z)-octadeca-10,13-dien-1-yl)-2-((9Z,12Z)-octadeca-9,12-dien-1-yl)-1,3-dioxolan-4 yl)methanamine also called D-Lin-K-DMA (pKa = 5.9), N,N-dimethyl-3-(2-((10Z,13Z)-octadeca-10,13-dien-1-yl)-2-((9Z,12Z)-octadeca-9,12-dien-1-yl)-1,3-dioxolan-4-yl)propan-1-amine also called DLin-KC3-DMA (pKa = 7.2), N,N-dimethyl-4-(2-((10Z,13Z)-octadeca-10,13-dien-1-yl)-2-((9Z,12Z)-octadeca-9,12-dien-1-yl)-1,3-dioxolan-4-yl)butan-1-amine also called DLin-KC4-DMA (pK = 7.3), SM-102 (pKa = 6.68), ALC-0315 (pKa = 6.09) (see e.g., US2017151333A1), L319 (pKa = 6.38), and YSK13-C3 (pKa = 6.45).

The pKa value of ionizable cationic lipids can be measured by commercial software such as that from ACD/Labs.

Phospholipids having a CPP > 1

The function of phospholipids having a CPP > 1 (*e.g.*, DOPE) in the LNVs of the present invention is to promote the non-lamellar structures of LNVs and to contribute to the stability and delivery efficacy of nanoparticles. Phospholipids of the present disclosure are those displaying a critical packing parameter (CPP) value > 1 (*i.e.*, an intrinsic curvature of < 0, inverse-cone shaped lipids) that usually promote non-lamellar phases (*i.e.*, hexagonal (HII) and/or sponge (L3) phases depending on the pH) in LNVs and subsequent fusion stalk in EVs. Without being so limited, the following phospholipids having a CPP > 1 can be used in accordance with the present disclosure: phosphatidylethanolamine (PE)-based lipids having a CPP > 1, monogalactosyldiacylglycerol (MGDG)-based lipids having a CPP > 1, monoglucosyldiacylglycerol (MGlcDG)-based lipids having a CPP > 1, diphosphatidylglycerol (DPG)-based lipids having a CPP > 1, phosphatidylserine (PS)- and phosphatidic acid (PA)-based lipids having a CPP > 1.

In specific embodiments, the at least one phospholipid having a CPP > 1 used in LNVs of the present disclosure is a phosphatidylethanolamine (PE)-based lipid having a CPP value > 1, in a more specific embodiment it is DOPE.

Non-ionic surfactants

The function of non-ionic surfactant displaying a CPP value < 1 (*e.g.*, KLP) in the LNVs of the present invention is to stabilize/contribute to form the non-lamellar structures of the LNV. In specific embodiments, the non-ionic surfactant acts as a membrane permeation enhancer. Non-ionic surfactants displaying an intrinsic curvature of > 0 and a CPP value < 1 can be used in LNVs of the present disclosure. Without being so limited, the following surfactants can be used in accordance with the present disclosure: polyethylene glycol esters of fatty acids (*e.g.*, polyethylene glycol 12-hydoxystearate (*e.g.*, a mixture of polyglycol mono- and di-esters of 12-hydroxystearic acid such as Kolliphor® HS15 (KLP))), polysorbates (*e.g.*, Tween® 20 and 80), polyethylene glycol aliphatic ethers such as those commonly known as Brij® 35 and 58, polyethylene glycol aliphatic esters, such as PEG stearates, and sorbitans (*e.g.*, Span® 60).

In specific embodiments, a polyethylene glycol esters of fatty acid such as the non-ionic surfactant KLP is used.

Spontaneous lipid curvature (CPP value)

The lipid geometry can be expressed by the CPP. CPP is related to the hydrocarbon chain volume (v) and length (I) and the interfacial area occupied by the polar head group (a) of lipids. Specifically, CPP is calculated by the following equation:

$$CPP = \frac{v}{l * a}$$

The volume difference between head group and tail blocks can be thought of as a measure of the lipid spontaneous curvature. The spontaneous curvature of a monolayer is dictated by a packing of the lipids in the membrane, i.e., the interaction of a lipid with its surrounding molecules. Thus, the spontaneous curvature of a monolayer may slightly differ in a bilayer membrane and a monolayer at an oil-water interface because the oil chains may differently interdigitate into the hydrophobic part of the monolayer than in the case of a bilayer. Lipids with large head groups and small tails (cone-like shaped, positive curvature, CPP < 1) will prefer to self-assemble into convex structures, such as micelles, whereas lipids with comparable volumes of their head group and tail blocks (cylindrical lipids, CPP ~ 1) will form planar bilayers or large vesicles, and lipids with small head groups and large tails (inverted cone-like, negative curvature, CPP > 1) will yield inverted aggregates, such as the inverted hexagonal phase. Different approaches (*i.e.,* X-ray diffraction and osmotic stress) can be used to measure the spontaneous curvature of lipids and their membranes. These methods and techniques are disclosed in *e.g.,* Sodt et al., 2013; Barragan Vidal et al., 2017; Rand et al., 1990; and Hamai et al., 2006.

LNV components proportions

The LNV of the present disclosure comprises a molar concentration of about 30 % to about 70 % (*e.g.,* about 35 % to about 70 %, about 35 % to about 65 %, about 35 % to about 60 %, about 40 % to about 60 %, about 40 % to about 65 %, about 40 % to about 70 %) of the at least one ionizable cationic lipid; of about 10 % to about 50 % (*e.g.*, about 10 % to about 45 %, about 10 % to about 40 %, about 10 % to about 38 %, about 10 % to about 35 %, about 10 % to about 30 %, about 10 % to about 25 %, about 10 % to about 20%; about 15 % to about 45 %, about 15 % to about 40 %, about 15 % to about 38 %, about 15 % to about 35 %, about 15 % to about 30 %, about 15 % to about 25 %, about 15 % to about 20%; about 18 % to about 45 %, about 18 % to about 40 %, about 18 % to about 38 %, about 18 % to about 35 %, about 18 % to about 30 %, about 18 % to about 25 %, about 18 % to about 20%) of the at least one phospholipid having a CPP > 1; and of about 10 % to about 50 % (*e.g.*, about 11 % to about 50 %, about 12 % to about 50 %, about 13 % to about 50 %, about 14 % to about 50 %, about 15 % to about 50 %, about 16 % to about 50 %, about 17 % to about 50 %, about 18 % to about 50 %, about 19 % to about 50 %, about 20 % to about 50 %; about 11 % to about 45 %, about 12 % to about 45 %, about 13 % to about 45 %, about 14 % to about 45 %, about 15 % to about 45 %, about 16 % to about 45 %, about 17 % to about 45 %, about 18 % to about 45 %, about 19 % to about 45 %, about 20 % to about 45 %; about 11 % to about 44 %, about 12 % to about 44 %, about 13 % to about 44 %, about 14 % to about 44 %, about 15 % to about 44 %, about 16 % to about 44 %, about 17 % to about 44 %, about 18 % to about 44 %, about 19 % to about 44 %, about 20 % to about 44 %; preferably about 11 % to about 43 %, about 12 % to about 43 %, about 13 % to about 43 %, about 14 % to about 43 %, about 15 % to about 43 %, about 16 % to about 43 %, about 17 % to about 43 %, about 18 % to about 43 %, about 19 % to about 43 %, about 20 % to about 43 %; about 22 % to about 42 %; about 11 % to about 35 %, about 12 % to about 35 %, about 13 % to about 35%, about 14 % to about 35 %, about 15 % to about 35 %, about 16 % to about 35 %, about 17 % to about 35 %, about 18 % to about 35 %, about 19 % to about 35 %, about 20 % to about 35 %; about 11 % to about 30 %, about 12 % to about 30 %, about 13 % to about 30%, about 14 % to about 30 %, about 15 % to about 30 %, about 16 % to about 30 %, about 17 % to about 30 %, about 18 % to about 30 %, about 19 % to about 30 %, about 20 % to about 30 %; about 11 % to about 25 %, about 12 % to about 25 %, about 13 % to about 25 %, about 14 % to about 25 %, about 15 % to about 25 %, about 16 % to about 25 %, about 17 % to about 25 %, about 18 % to about 25 %, about 19 % to about 25 %, about 20 % to about 25 %) of the at least one non-ionic surfactant displaying a CPP value < 1. The molar concentration is selected so as to enable useful loading efficiencies of drug or diagnostic agent (*e.g.,* nucleic acid) in the LNVs. When the LNV of the present disclosure comprise only the at least one ionizable cationic lipid, the at least one phospholipid having a CPP > 1; and the at least one non-ionic surfactant displaying a CPP value < 1, the sum of the molar concentrations of each of these components amounts to 100%.

Drug

Drug(s) as used herein refers to any pharmacologically active molecule or mixture of molecules (including non approved active pharmaceutical ingredients (APIs)). As used herein, mixtures of molecules display pharmacological activity. Without being so limited, biological extracts such as plant or animal extracts are encompassed by the term mixture of molecules, and thereby by the term drug. Drugs that can be incorporated into the device of the present disclosure can be low molecular weight APIs (< 1000 Da) or large molecules (> 1000 Da) such as polysaccharides, peptides, proteins, nucleic acids (*e.g.,* RNA (such as siRNA, mRNA, etc.), DNA), proteolysis-targeting chimeras (PROTACs), antibody-drug conjugates, and vaccine antigens.

Diagnostic agent

Diagnostic agents as used herein refer to any pharmaceutical product used as part of a diagnostic test, such as radiopharmaceuticals, contrast agents for use in imaging techniques including X-rays, magnetic resonance imaging (MRI) and ultrasound (US), compounds used in diagnostic tests that do not involve radioisotopes or imaging techniques, various stains/markers (*i.e.,* stains used in detection of malignant cells), antibodies (*i.e.,* monoclonal antibodies) and nucleic acid-based probes. In specific embodiments, the diagnostic agent is a negatively charged molecule (*e.g.,* nucleic acid containing diagnostic agent) or a hydrophobic molecule.

Drug and diagnostic agent concentration

LNVs of the disclosure may be used to entrap drugs or diagnostic agents. When the drug or diagnostic agent is a nucleic acid, the proportion of ionizable cationic lipid in the LNV vs. the nucleic acid drug or diagnostic agent in the nucleic acid-loaded LNV of the present disclosure (*i.e.,* N/P ratio) is $\geq$ than about 1 (*e.g.,* $\geq 2$, $\geq 3$, $\geq 4$, $\geq 5$, between 6 and 300, or between 6 and 200, or between 6 and 150 or between 6 and 100 or between 6 and 50).

Drug or diagnostic formulation

LNVs are prepared by mixing an aqueous phase (*e.g.,* saline, pure water) at an acidic pH and an organic medium (*e.g.,* ethanol). Hydrophilic drugs will be dissolved in the aqueous phase prior to the entrapment into LNVs, whereas hydrophobic drugs will be dissolved in the organic phase prior to the loading into LNVs.

Subject

As used herein the term "subject" is meant to refer to any animal, such as a mammal, including a human, dog, cat, pig, cow, monkey, cattle, horse, etc. In a particular embodiment, it refers to a human, in specific embodiments to an adult subject.

Method of making drug or diagnostic agent loaded LNVs

LNVs of the present disclosure are prepared by mixing (i) an organic phase at least one ionizable cationic lipid, at least one phospholipid having a CPP > 1, at least one non-ionic surfactant displaying a CPP value < 1 dissolved in a solvent; and (ii) an aqueous phase. In the mixing process, the aqueous-to-organic solvent volumetric flow rate ratio is between 0.5:1 and 5:1. In a more specific embodiment it is of 1.5:1. When producing a hydrophobic drug (or diagnostic agent) loaded LNV, the hydrophobic drug (or diagnostic agent) is included in the organic phase, and when producing a hydrophilic drug (or diagnostic agent) loaded LNV (*e.g.,* nucleic acid), the hydrophilic drug (or diagnostic agent) is included in the aqueous phase.

Mixing methods used to produce LNVs of the present disclosure include any microfluidic devices. Without being so limited, it includes any microfluidic chip designs, including micromixer, hydrodynamic flow focusing chip, T-junction chip, chaotic micromixer, bifurcating mixer and baffle mixer. In a particular embodiment, it is a chip formed by a Y-shaped inlet design. In specific embodiments, the mixing device used is a microfluidic device employing a high-precision syringe pump. In specific embodiments, the microfluidic device has a total flow rate between 0.001 mL/min and 100 mL/min, preferably, the flow rate is of about 0.05 mL/min; and/or the microfluidic device has a volumetric flow rate ratio of between 0.5:1 and 5:1; and/or the microfluidic device has a microfluidic channel depth of between 1 $\mu$m and 500 $\mu$m, preferably about 100 $\mu$m, channel inlets width between 1 $\mu$m and 500 $\mu$m, preferably about 100/200 $\mu$m, channel mixer width of between 1 $\mu$m and 500 $\mu$m, preferably about 200 $\mu$m and channel outlet width between 1 $\mu$m and 500 $\mu$m, preferably about 200 $\mu$m.

Mixing methods also include ethanol injection, thin film evaporation, reversed-phase evaporation, high-pressure homogenization (HPH), high shear homogenization, sonication, microemulsion formation, and any other approach that leads to the production of lipid-based particles.

pH-inducible structure-switching non-lamellar LNVs of size 20 to 200 nm, in specific embodiments about ~ 100 nm, formed by hexagonal (HII) phases at acidic pH and sponge (L3) phases at neutral pH are produced (FIGs. 1A, 1G-H, 2A).

Organic phase solvent

The organic solvent used in the present disclosure is any biocompatible solvent in which the ionizable cationic lipid, and phospholipid having a CPP > 1 are soluble. Without being so limited, it includes alcohol (*e.g.,* ethanol), oils (*e.g.,* fluorinated), and chloroform. In specific embodiments, the organic phase solvent is ethanol. The concentration of organic solvent used is sufficient to dissolve the lipids. In specific embodiments, the concentration of lipids in the solvent is between 10 $\mu$g of lipids per mL of solvent to 1 g lipids per mL of solvent. In specific embodiments, the concentration of lipids in the solvent is between 1 mg of lipids per mL of solvent to 20 mg of lipids per mL of solvent., or between 5 mg of lipids per mL of solvent to 15 mg of lipids per mL of solvent, or between 10 mg of lipids per mL of solvent to 15 mg of lipids per mL of solvent.

Aqueous medium

An aqueous medium is used in the production of the LNVs (aqueous phase) and in the hybridization step between the LNVs and the EVs. The aqueous medium used in the present disclosure is any aqueous medium, including pure water or saline. The aqueous medium may be of diverse compositions, pH and osmotic strength.

When the drug or diagnostic agent to be loaded in the LNVs is negatively charged (*e.g.,* nucleic acid such as RNA), the pH of the aqueous medium used in the production of LNVs (*e.g.,* nucleic acid loaded LNVs) may have a pH ranging from about 4 to about 6, *e.g.,* 4, 4.5, 5, 5.5, 6, and preferably between about 4 and 6. In specific embodiments, sodium acetate buffer (pH 4) is used. The pH may be achieved by adding buffer components (*e.g.,* acetate buffer, sodium acetate, citrate buffer, sodium citrate or sodium citrate phosphate buffer), and/or an acid or base (*e.g.,* hydrochloric acid and/or sodium hydroxide) for pH adjustments. When the drug or diagnostic agent to be loaded in the LNVs is neutral, the pH of the aqueous medium may also be higher than 6 and up to 10. After the formation of the LNVs, the aqueous solution may be neutralized to a pH 7.4 with *e.g.,* a phosphate buffer (such as PBS) or HEPES-buffered saline (HEBS) or a Tris buffer.

The aqueous solution containing the LNVs can be formulated for storage for future use or adapted for the step of hybridization with the EVs by increasing the pH to about 7 or more, and up to about 10. If the aqueous solution containing the LNVs has already been neutralized (*e.g.,* with PBS), it may be more precisely adapted with an acid or base (*e.g.,* hydrochloric acid and/or sodium hydroxide).

The aqueous medium used for the hybridization step may further contain osmotic agents to achieve an isotonic solution with the EVs. EVs will generally have an osmolarity of 200-500 mmOsm/kg. Osmotic agents such as sodium chloride, glucose, gelatin, xylitol, sorbitol, mannitol, glucose polymer (*e.g.,* icodextrin), or amino acids) may be used.

The pH of the aqueous medium used in the hybridization step is higher than 6 and up to about 10, higher than 7, higher than 7.1, higher than 7.2, higher than 7.3, or between about 7.4 and about 10.

EVs

The generic term "EVs" describes herein native EVs and synthetic or bioengineered EVs prepared to mimic native EVs (see *e.g.,* exoIL-12™, exoSTING™, exoASO™-STAT6, AGLE-102™, ExoAAV, OmniSome, ILB-202)). "Native EVs" can be defined as heterogeneous populations of membranous vesicular structures originating from any eukaryotic or prokaryotic cell or tissue. They can be classified depending on their characteristic features such as size, density and composition or biogenesis pathway, *e.g.,* small EVs ($\leq$ 200 nm) from mammalian cells are mostly of endosomal origin and are often referred to as exosomes. Medium or large EVs (> 200 nm) are often formed via plasma membrane budding and are referred to as exosomes/microvesicles or by shedding of apoptotic bodies during cell death. (Théry et al., 2018).

The term EVs further refers to a fraction of EVs as described above and/or to an extruded/homogenized form of native EVs and to an extruded form of whole cells. Extrusion/homogenization is conducted by any known method *e.g.,* extrusion through nano-/micro-sized filter pores using high-pressure extruders or centrifugation setups, as well as mechanical homogenization/disruption of cells/EVs *e.g.,* via sonication, nitrogen cavitation, freeze-thaw cycles or Dounce homogenizer.

ssEVs of the present disclosure retain at least a portion of EV surface/membrane protein functionality, EV inherent bioactivity (*e.g.*, regenerative activity, anti-inflammatory activity, angiogenesis, cardioprotective, and homing/-targeting properties (*i.e.,* ability to recognize specific tissues/cells).

Method of isolating EVs

EVs may be isolated from biological samples with known methods such as centrifugation-based techniques (*e.g.,* ultracentrifugation and density gradient centrifugation), affinity-based techniques (*e.g.,* protein-based affinity (such as antibody capture or MagCapture™ kit) or membrane-based affinity (such as ExoEasy™ kit)), precipitation-based techniques (*e.g.,* ExoQuick™ kit), microfluidic-based techniques, filtration-based techniques (*e.g.,* tangential flow filtration and ultrafiltration), and size exclusion chromatography as described in Wu et al., 2023. In a specific embodiment, EVs are isolated using a combination of differential centrifugation, microfiltration and ultracentrifugation.

As used herein the term "biological sample" from which EVs for use in the present disclosure may be isolated includes any cells and tissues derived from eukaryotes and prokaryotes. It includes cultured animal (mammalian such as human) cell line or primary cells belonging to any tissue/organ (*e.g.,* lung, brain, pancreas, tumor/cancer, liver, bone marrow, blood, etc.). Any human/mammalian biofluid *(*e.g., blood, CSF, serum, urine, (breast) milk, saliva, synovial fluid, bile, ascites fluid, amniotic fluid semen, excrement). Any cultured bacterial strain (gram-positive and gram-negative). Any EV from fungi and plants.

Method of making hybrid EV particles (ssEVs)

The LNVs (*e.g.,* drug-loaded, such as nucleic acid loaded, diagnostic agent loaded) are mixed (*e.g.,* incubated) with EVs in an aqueous medium (see above, *e.g.,* (PBS)) with the EVs at a temperature between 4 and 60 °C, and at a pH between larger than 6 and up to 10, between 6.1 and 10, between 6.2 and 10, between 6.3 and 10, between 6.4 and 10, between 6.5 and 10, between 6.6 and 10, between 6.7 and 10, between 6.8 and 10, between 6.9 and 10, between 7 and 10, between 7.1 and 10, between 7.2 and 10, between 7.3 and 10, between 7.4 and 10, between 7.5 and 10, between 6.1 and 9, between 6.2 and 9, between 6.3 and 9, between 6.4 and 9, between 6.5 and 9, between 6.6 and 9, between 6.7 and 9, between 6.8 and 9, between 6.9 and 9, between 7 and 9, between 7.1 and 9, between 7.2 and 9, between 7.3 and 9, between 7.4 and 9, and between 7.5 and 9. The mixing is conducted for a duration of between 1 second and 48 hours (e.g., 5, 10, 15, 20, 30, 40, 50, 55 seconds; 1, 2, 3, 4, 5, 6, 10, 15, 20, 25, 30, 55 minutes, 1, 2, 3, 4, 5, 6, 7, 8, 9, 10, 11, 12, 13, 14, 15, 20, 24, 30, 36 or 42 hours, and any range thereof).

ssEVs

The ssEVs are the result of the LNVs and EV fusion (see *e.g.,* FIGs. 2C-E, 2G-I, FIG. 3E and 3G). In specific embodiments they are produced by the method of the present disclosure. In other specific embodiments, they contain essentially all of the components, partially or completely mixed, of an LNV of the present disclosure and of EVs as described herein. As used in this context, the terms "essentially all of the components" is meant to denote that while a large majority (at least 70%, at least 75%, at least 80%, at least 90%, at least 95%, 96%, 97%, 98% or 99%) of the components of each of the LNV and of the EVs are in the ssEVs, they do not necessarily contain all of them (*e.g.,* because of the method of producing them). As used in this context, the terms "partially or completely mixed" is meant to encompass the ssEVs as it exists during all phases of the fusion (see *e.g.,* FIGs. 2C-2E and 3F).

The average size of ssEVs in accordance with the present disclosure is between about 20 nm and 2000 nm (dependent on *e.g.,* EV source, extrusion, etc.). In specific embodiments the average size is about 20 nm ~ 500 nm, 20 nm ~ 450 nm, 20 nm ~ 400 nm, 20 nm ~ 350 nm, 20 nm ~ 300 nm, 20 nm ~ 250 nm, about 50 nm ~ 250 nm, about 75 nm ~ 250nm, about 100 nm ~ 250 nm, about 125 nm ~ 250 nm, about 130 nm ~ 250 nm, about 140 nm ~ 250 nm, about 150 nm ~ 250 nm, about 160 nm ~ 250 nm, about 20 nm ~ 225 nm, about 50 nm ~ 225 nm, about 75 nm ~ 225 nm, about 100 nm ~ 225 nm, about 125 nm ~ 225 nm, about 130 nm ~ 225 nm, about 140 nm ~ 225 nm, about 150 nm ~ 225 nm, about 160 nm ~ 225 nm, about 20 nm ~ 215 nm, about 50 nm ~ 225 nm, about 75 nm ~ 215 nm, about 100 nm ~ 215 nm, about 125 nm ~ 215 nm, about 130 nm ~ 215 nm, about 140 nm ~ 215 nm, about 150 nm ~ 215 nm, about 160 nm ~ 215 nm, about 20 nm ~ 200 nm, about 50 nm ~ 200 nm, about 75 nm ~ 200 nm, about 100 nm ~ 200 nm, about 125 nm ~ 200 nm, about 130 nm ~ 200 nm, about 140 nm ~ 200 nm, about 150 nm ~ 200 nm, and about 160 nm ~ 200 nm.

The present method allows for the *in situ* (*i.e.,* at the point of care) production of ssEVs, before administration to a subject in need thereof.

Composition/formulation

Pharmaceutically acceptable excipients include one or more of cryoprotectants (*e.g.,* sucrose, dextrose, trehalose, or synthetic polymers such as poloxamers and polyvinylpyrrolidone (PVP), etc.), osmotic agents (*e.g.,* dextrose, lactulose, sorbitol, magnesium hydroxide, magnesium citrate, sodium chloride, potassium chloride, polyethylene glycol (PEG), etc.), salt(s) (*e.g.,* sodium phosphate, potassium phosphate, tris, sodium citrate, etc.).

LNV composition/formulation

LNVs can be mixed with one or more pharmaceutically acceptable excipients to form a composition (liquid or solid) and e.*g.,* stored for future use. For example, they may be freeze-dried in the presence of at least one of (i) one or more cryoprotectants (*e.g.,* sucrose, dextrose, trehalose, or synthetic polymers such as poloxamers and PVP), (ii) one or more osmotic agents such as *e.g.,* dextrose, PEG, lactulose, sorbitol, magnesium hydroxide, magnesium citrate, sodium chloride and potassium chloride), and (iii) one or more salt(s) (*e.g.,* sodium phosphate, potassium phosphate, tris, sodium citrates) before resuspension with an aqueous medium, or stored as an aqueous suspension at a temperature ranging from - 80 to 8°C.

In specific embodiments, at least one of cholesterol and PEG lipids (PEGylation) could be added to LNVs (*e.g.,* DSPE-PEG MW = 2 kDa, DMG-PEG, MW = 2 kDa).

ssEV composition/formulation

ssEVs can be mixed with one or more pharmaceutically acceptable excipients to form a composition (liquid or solid) and *e.g.,* stored for future use. For example, they may be freeze-dried in the presence of cryoprotectants (*e.g.,* sucrose, dextrose, trehalose, or synthetic polymers such as poloxamers and PVP) and salt compositions (*e.g.,* sodium phosphate, potassium phosphate, tris, sodium citrates, sodium chloride and potassium chloride) before resuspension with an aqueous medium, or stored as an aqueous suspension at a temperature ranging from - 80 to + 8 °C.

They can also be embedded in polymer matrices (*e.g.,* as gels, implants or devices) or classical pharmaceutical formulations (*e.g.,* ointments, capsules, pre-filled syringes, etc.).

In specific embodiments, at least one of cholesterol and PEG lipids (PEGylation) could be added to ssEVs could be pegylated to *e.g.,* increase their stability (*e.g.,* DSPE-PEG, MW = 2 kDa, DMG-PEG, MW = 2 kDa). ssEVs may alternatively be formed *in situ* (at point of care location) before injection to a subject in need thereof.

Kit

Also within the scope of the invention are kits comprising (a) at least one LNV of the present disclosure or a composition (liquid or solid *e.g.,* LNV powder (*e.g.,* lyophilized)) comprising at least one LNV and at least one pharmaceutically acceptable excipient; and (b) (i) a solution for hydrating the at least one LNV or composition thereof (*e.g.,* prior to its use), optionally with at least one pharmaceutically acceptable excipient; (ii) a drug or diagnostic agent (*e.g.,* for loading in LNV or for use as control); (iii) a composition (liquid or solid) comprising EVs and at least one pharmaceutically acceptable excipient; (iv) instructions for using the at least one LNV or composition thereof (*e.g.,* instructions for the production of ssEVs from LNVs and/or instructions for administration of ssEVs); or (v) a combination of at least two of (i) to (iv). In a specific embodiment, the kit comprises (a) at least one LNV (unloaded or loaded with drug or diagnostic agent) in a solid form (*e.g.,* lyophilized) or a composition in a solid form (e.g., lyophilized) comprising the at least one LNV and at least one pharmaceutically acceptable excipient (*e.g.,* at least one cryoprotectant); and (b) (i) a solution for hydrating the at least one LNV or composition, the solution containing a least one pharmaceutically acceptable excipient (*e.g.,* at least one of osmotic agent and salt, to provide isotonic environment to the LNVs). In another specific embodiment, the kit further comprises (b) (ii) a drug or diagnostic agent (*e.g.,* for loading in LNV or for use as control).

Also within the scope of the invention are kits comprising (a) at least one ssEV of the present disclosure, or a composition (liquid or solid) comprising the at least one ssEV and at least one pharmaceutically acceptable excipient; and (b) (i) a solution for hydrating the at least one ssEV or composition thereof (prior to its use); (ii) instructions for the use of these ingredients (*e.g.,* instructions for administration of at least one ssEV or composition thereof); or (iii) a combination of (i) and (ii).

Kits typically include a label indicating the intended use of the contents of the kit. The term label includes any writing, or recorded material supplied on or with the kit, or which otherwise accompanies the kit. The kit may further comprise one or more container(s), reagent(s), administration device(s). An example of such a kit comprises (i) a first composition (liquid or solid) comprising LNVs contained in a first container or in a first compartment of the first container; and (ii) a second composition (liquid or solid) comprising EVs and at least one pharmaceutically acceptable excipient in a second container or in a second compartment of the first container. Prior to use, the first and second compositions could be mixed by allowing the content of the first and second compartments to be joined by *e.g.,* breaking a wall separating these compartments. After the formation of the ssEVs in the mixture, the resulting solution can be administered to a subject in need thereof.

Uses

The LNVs (*e.g.,* drug or diagnostic agent loaded) of the present disclosure can be included in a commercial kit for use to produce the ssEVs. Such ssEVs can be used in *in vitro* and *in vivo* studies or for clinical (*e.g.,* therapeutic or diagnostic) purposes.

Route of administration

The ssEVs of the present disclosure can be administered parenterally, topically or orally.

Subject

As used herein the terms "subject" or "subject in need thereof" refer to a subject who would benefit from receiving an effective amount of the ssEVs or composition thereof of the present invention. It refers to an animal, a mammal and to a human in a specific embodiment. The ssEVs and compositions of the present invention may also be used for veterinary applications and be used in pets or other animals (*e.g.,* pets such as cats, dogs, horses, etc.; and cattle, fishes, swine, poultry, etc.).

The use of the terms "a" and "an" and "the" and similar referents in the context of describing the invention (especially in the context of the following claims) are to be construed to cover both the singular and the plural, unless otherwise indicated herein or clearly contradicted by context.

The terms "comprising", "having", "including", and "containing" are to be construed as open-ended terms (i.e., meaning "including, but not limited to") unless otherwise noted.

Recitation of ranges of values herein are merely intended to serve as a shorthand method of referring individually to each separate value falling within the range, unless otherwise indicated herein, and each separate value is incorporated into the specification as if it were individually recited herein. All subsets of values within the ranges are also incorporated into the specification as if they were individually recited herein.

All methods described herein can be performed in any suitable order unless otherwise indicated herein or otherwise clearly contradicted by context.

The term "about" has its ordinary meaning. In embodiments, it may mean plus or minus 10% of the numerical value qualified. Herein, the term "approximately" has its ordinary meaning. In embodiments, it may mean plus or minus 10% of the numerical value qualified.

The use of any and all examples, or exemplary language (e.g., "such as") provided herein, is intended merely to better illuminate the invention and does not pose a limitation on the scope of the invention unless otherwise claimed.

No language in the specification should be construed as indicating any non-claimed element as essential to the practice of the invention.

The present disclosure is illustrated in further details by the following non-limiting examples.

EXAMPLE 1: Material and method

Preparation of LNVs

*Unloaded LNVs*

LNVs and nanoparticles were prepared by mixing an organic phase comprising the components listed in Table I below at the molar concentrations (mol %) listed in Table I below, in an ethanol solvent with an aqueous phase comprising an acetate buffer at pH 4 (see FIG. 1A for LNVs of Examples 2-9). The method for making the nanoparticles of Examples 10-12 is as presented in FIG. 1A except that the organic phase contains the components listed in each of the corresponding columns of Table I.

Table I: Components of LNVs and nanoparticles used in Examples herein

| Components | Concentration (mol %) | | | | | |
|---|---|---|---|---|---|---|
| MC3 | 40 | 50 | 60 | 60 | 60 | |
| DOPE | 18 | 18 | 18 | | 18 | 18 |
| DOPC | | | | 18 | | |
| KLP | 42 | 32 | 22 | 22 | | 22 |
| DOTMA | | | | | | 60 |
| MPPC | | | | | 22 | |
| Examples Nos. | 2 | 2 | 2-9 | 10 | 11 | 12 |

The mixing was performed by introducing each phase in a microfluidic device through separate syringes (see FIG. 1A). A chip that allows diffusion mixing and is based on a Y-shaped inlet design with channel depth 100 $\mu$m, channel width inlets 100/200 $\mu$m, channel width mixer 200 $\mu$m and channel width outlet 200 $\mu$m was used (Fluidic™ 186 - Micro Mixer purchased from Microfluidic ChipShop, Germany). In the mixing process, the aqueous phase-to-organic phase volumetric flow rate ratio was set to 1.5:1 and a high-precision syringe pump was used with a total flow rate of 0.05 mL/min.

The produced LNVs were successively purified from the organic solvent *via* dialysis with a Slide-A-Lyzer™ Mini Device (MWCO = 20 kDa) against 1000-fold volume excess of PBS at pH 7.4 or HEBS (20 mM HEPES, 150 mM NaCl) at pH 7.4. This led to the production of LNVs at pH 7.4. For the production of LNVs at pH 5, hydrochloric acid was added to the sample. The pH value of all formulations was carefully checked with a pH meter.

*siRNA-loaded LNVs*

The siRNA-loaded LNVs were prepared as were the unloaded LNVs except that siRNA was added to the aqueous phase after pH adjustment.

The siRNA against green fluorescent protein (GFP-siRNA) with a nucleotide sequence 5'-GAA CUU CAG GGU CAG CUU GGG-3' (SEQ ID NO: 1) (3' overhang: sense (5'-3') dGdG; antisense (5'-3') dTdT); MW = 13365.1 g/mol) at concentrations of 5.7, 11.4, 22.8, 45.6 $\mu$M to obtain N/P ratios of 50, 25, 12 and 6, respectively. The GFP-siRNA was custom synthesized by Microsynth AG (Switzerland).

For the nanoparticle flow cytometry (NanoFCM) experiments, the siRNA used was either FAM-labeled siRNA (MISSION® siRNA Fluorescent Universal Negative Control, 6-FAM, Sigma-Aldrich, USA); MW = 13854 g/mol) at a N/P ratio of 25; or GFP-siRNA-loaded LNVs labeled by replacing one mol % of DOPE with Atto 488-labeled DOPE.

For *in vitro* knock down experiments, LNVs were additionally loaded with a scrambled, control siRNA sequence as negative control (CTRL-siRNA with a nucleotide sequence 5'-UAA GGC UAU GAA GAG AUA CTT -3' (SEQ ID NO: 2) (3' overhang: sense (5'-3') dTdT; antisense (5'-3') dTdT; MW = 13270.0) at a N/P ratio of 25. The control siRNA was custom synthesized by Microsynth AG (Switzerland).

*mRNA-loaded LNVs*

The LNVs were prepared as described above except that mRNA was added to the aqueous phase after pH adjustment.

mRNA: Firefly luciferasemRNA (FLuc-mRNA (TriLink BioTechnologies, USA); MW ~ 637715.0 g/mol or Cy-5-labeled firefly luciferase mRNA (Cy5-FLuc-mRNA (Cellerna Bioscience, Germany)); MW ~ 785980.0 g/mol) at the concentration of 0.25 $\mu$M to obtain an N/P ratio of 25.

Nanoparticles produced without MC3

Nanoparticles were prepared by using the same protocol as LNVs, as described above, except that the organic phase contained DOPE and KLP at the molar concentrations of 48 % and 52 %.

Gel electrophoretic mobility shift assay (EMSA)

For EMSA experiments 0.1 $\mu$g of GFP-siRNA loaded in LNVs or free GFP-siRNA were mixed with or without 1 % (v/v) Triton™ X-100 and loaded on a 0.5 % (w/v) agarose gel. The gel was run for 30 min at 70 V in 0.5 $\times$ TAE buffer (Tris-base 20 mM, 10 mM acetic acid and 0.5 mM EDTA), stained for 45 min with SYBR™ Gold Nucleic Acid Gel Stain (Thermo Fischer Scientific, USA) and imaged with a ChemiDoc™ MP Imaging System (Bio-Rad, USA). Similarly, EMSA experiments were performed after preincubation with 5 $\mu$g/mL RNase A for 1 h at 37 °C (with/without Triton™ X-100) to assess the protection of the siRNA cargo from nuclease-mediated degradation.

Cell culture

For EV production, the bone marrow-derived mesenchymal stem cell (MSC) line HS-5 was cultured on microcarriers in a 3D culture setup. Therefore, 118 $\times$ 10$^6$ HS-5 cells were seeded on 4.5 g of Corning™ Enhanced Attachment microcarriers (Corning, USA) in a total volume of 300 mL serum-containing DEMEM inside 500 mL Corning™ Reusable Glass Spinner Flasks (Corning, USA). An intermitted stirring protocol (5 min on at 30 rpm and 60 min off) was applied before the impeller speed was set to 35 rpm in continuous mode. After 48 h, microcarriers were washed twice with PBS and 400 mL fresh, serum- and phenol red-free medium was added to the spinner flasks. Cell culture supernatant was harvested after 48 h of culture in serum-free medium.

Isolation of EVs from cell culture supernatant

EVs were isolated from cell culture supernatant using a combination of centrifugation, microfiltration and ultracentrifugation steps. First, the supernatant was centrifuged at 500 and 2,000 $\times$ g for 5 min each and 10,000 $\times$ g for 20 min using a Sorvall™ LYNX 6000 centrifuge equipped with a Fiberlite™ F12-6 $\times$ 500 LEX Fixed Angle Rotor (both Thermo Fisher Scientific, USA). The clarified cell culture supernatant was then filtered through a 0.2-μm membrane before an ultracentrifugation step (70 min at 4 °C and 100,000 × g) was performed using an Optima™ XE-90 ultracentrifuge (UC) (Beckman Coulter, USA) and a Type 45 Ti Fixed-Angle Titanium Rotor. After the first ultracentrifugation run, EV pellets were washed with PBS or HEBS and pooled together. Another centrifugation step with identical conditions was performed and the EV pellet was suspended in ice-cold PBS/HEBS and stored at -20 °C.

## Hybridizing nucleic acid (NA)-loaded LNVs with EVs - ssEV formation

The different RNA-loaded LNVs (i.e., siRNA and mRNA as described above) were mixed with MSC-derived EVs in an aqueous medium (either PBS or HEBS at pH 5, 6, 7.4 or 9) at different EV: LNV particle number ratios (4:1, 1:1 and 1:4), time points (5 and 30 min, 1 and 4 h), temperatures (4, 25 and 37°C) and pH values (5, 6, 7.4 and 9). To achieve specific pH values used, the pH of the LNVs was carefully adjusted by the addition of hydrochloric acid and/or sodium hydroxide, if necessary, and checked with the pH meter. More particularly, a 62.5 mM hydrochloric acid or sodium hydroxide solution was used to respectively decrease or increase the pH. The pH of the samples was carefully checked each time using a pH electrode InLab Micro™ (Mettler Toledo, USA).

## HeLa cell viability

Cell viability was determined using the CellTiter 96® AQueous One Solution Cell Proliferation Assay (MTS) (Promega, Switzerland). In brief, HeLa cells (2,500 cells per well in a total volume of 200 μL complete DMEM) were seeded in a 96-well plate and incubated for 24 h at 37 °C. Two μL of LNVs or GFP-siRNA-loaded LNVs, at specific final lipid concentrations (ranging from 12.5 to 200 μg mL$^{-1}$, df: 1:2) were then added to the wells and incubated with the cells for 4 and 24 h. As a negative control, cells received 2 μL PBS. As a positive control, cells received 0.5% (v/v) Triton™ X-100. Then, the medium was removed, cells were gently washed with PBS and 100 μL complete DMEM was added. Twenty μL MTS reagent was added and HeLa cells were incubated for 1 h at 37 °C. Absorbance was recorded at 490 nm, using a Tecan Spark® and an Infinite™ M200 plate reader (both Tecan, Switzerland). Results were processed using i-control™ 2.0 Software. Three independent batches of all formulations were tested (n = 3).

## Cryogenic transmission electron microscopy (cryo-TEM)

Three μL of ssEV, LNV or EV suspensions in PBS were added on glow-discharged (30 s in a Emitech™ K100X glow discharge system, Quorum Technologies Ltd., UK) 300-mesh lacey carbon-coated copper grids. A Vitrobot™ Mark II (Thermo Fisher Scientific, USA) was used to vitrify the samples in a mixture of liquid ethane and propane. Sample excess was removed by controlled botting and the grids were then transferred on a Gatan™ cryo-holder and imaged with a Tecnai™ F20 Cryo microscope (Field Electron and Ion Company, USA) operated at a 200 kV acceleration voltage in the bright field mode and maintained at -180 °C during observation. Micrographs were recorded under low-dose conditions (<500 electrons nm-2) using a Falcon II™ 4K Direct Electron Detector (Field Electron and Ion Company, USA).

## Small-angle X-ray scattering (SAXS)

A volume of 20 μL of samples was carefully added to borosilicate glass capillary tubes. The capillary tubes, with a length of 80 mm and a wall thickness of 0.01 mm, were loaded using hypodermic stainless-steel needles. To ensure a hermetic seal, the upper part of each capillary was meticulously closed using ultra-high vacuum 2-K-epoxy resin adhesive (1-part resin to 1-part hardener, v/v, UHU plus, Germany) over the course of two consecutive one-hour sessions. Subsequently, the capillaries were inserted into a specialized sample holder before being loaded into the instrument for further analysis. SAXS measurements were performed using an in-house SAXS setup (Xeuss™ 3.0, Xenocs, Grenoble, France) equipped with a Genix™ 3D source (Xenocs). The X-ray energy was 50 kV (A = 1.54 Å, Cu K$_\alpha$), and the photon flux at the sample position was > 10$^{-7}$ counts/sec. The beam size on the sample was ca. 0.5 × 0.5 mm$^2$ FWHM. The sample to-detector distance, calibrated using a silver behenate standard, was 500 mm and the scattered signal was collected on a Dectris Eiger 1 M detector (1028 × 1062 pixels, pixel size 75 × 75 μm$^2$ Dectris Ltd., Baden, Switzerland). In total, the signal was recorded for > 8400 s. SAXS data are shown in the q-range from 0.016 to 0.69 Å-1, corresponding to real length scales from 1.0466 to 39.25 nm. Detector data were masked, azimuthally integrated, normalized to absolute intensity (cm$^{-1}$) and background-subtracted using XSACT (Xenocs) 2.6 software package.

The integrated intensity I(q) is plotted against the magnitude of the scattering vector q, as defined by following equation:

$$q = \frac{4\pi \sin \theta}{\lambda}$$

## Dynamic and static light scattering (DLS/SLS) experiments

The hydrodynamic size ($d_h$ in nm) and zeta potential ($\zeta$-pot in mV) of LNVs were measured with DLS using a Malvern Zetasizer™ Advance Pro instrument (Malvern Panalytical, UK) operated at a scattering angle of 179 ° at 25 °C. DLS and SLS time course experiments were performed with a DynaPro™ Plate Reader III (Wyatt Technology, USA) under temperature-controlled conditions (4°C, 25 °C and 37 °C) in sealed 384-well microplates (Aurora Microplates, USA). The microplate was pre-incubated with 30 µL of LNV suspensions ($\sim 8 \times 10^8$ particles/mL) for $\sim$ 30 min until a stable reading of the particle concentration was reached. An equal particle amount (except for ratio experiments) of EVs was then added and the measurement (particle concentration and $d_h$) was continued for a time course of 1 h.

## Assessing CD73 and DPP4 enzymatic activity

EVs were either subjected to a treatment with LNVs to form ssEVs of the present disclosure or transfected with the Exo-Fect™ Exosome Transfection Kit (System Biosciences, USA) prior to measuring the enzymatic activity. For measuring the activity of the EV membrane protein CD73, an absorbance-based malachite green assay was employed. Therefore, 0.1 µg of EV proteins from the differently tested conditions (in HEBS, pH 7.4) were suspended in 60 µL of 10 mM Tris buffer (pH 7.4) and 30 µL of 800 µM AMP stock solution in 10 mM Tris buffer were added to reach a final concentration of 240 µM. The mixture was then incubated for 10 min at 25 °C and the enzymatic reaction was stopped by adding 40 µL color reagent (0.034 % (w/v) malachite green, 1.55 % (w/v) ammonium molybdate tetrahydrate, 0.0625 % (v/v) polysorbate 20) to the wells. The plate was incubated for 1 h at 25 °C and the absorbance was measured at 620 nm with the Infinite™ M200 plate reader. The background subtracted absorbance values were normalized to the values obtained from EVs that were subjected to identical hybridization conditions.

For assessing the DPP4 enzymatic activity after hybridization, a commercially available DPPIV-Glo™ Protease Assay Kit (Promega, Switzerland) was used according to manufacturer instructions. Briefly, 0.08 µg of EV proteins in 25 uL of HEBS (pH 7.4) were mixed with an equal amount of DPPIV-Glo™ Reagent and incubated for 30 min at 25 °C. Luminescence was measured with an Infinite™ M200 Pro plate reader (Tecan, Switzerland) and the values were normalized with the values obtained from EVs that were subjected to identical hybridization conditions.

## Nanoparticle flow cytometry (NanoFCM) analysis

EVs were labeled with either CellTrace™ Far Red (Thermo Fisher Scientific, USA) or with 5(6)-carboxyfluorescein diacetate N-succinimidyl ester (CFDA-SE) (Chemodex AG, Switzerland) dye according to manufacturer instructions with certain modifications. Briefly, EVs were mixed with 5 µM of the respective dyes and incubated overnight at 4 °C. Subsequently, the mixtures were incubated at 37 °C for 15 min to allow the dye to undergo acetate hydrolysis. Unbound dye was subsequently removed by dialysis against a 1000-fold access of PBS at 4 °C overnight using the Pur-A-Lyzer™ Mini 6000 Dialysis kit (Sigma-Aldrich, USA). Fluorescently labeled LNVs (FAM-siRNA, Cy5-FLuc-mRNA and Atto 488 DOPE) and labeled EVs where then mixed at a particle ratio of 1:1 at 4 °C or 37 °C (30 min), and ssEV formation was assessed using a Flow NanoAnalyzer™ N30 (NanoFCM Co., Ltd, UK) instrument.

For control experiments with the Exo-Fect™ Exosome Transfection Kit, an equal amount of FAM-siRNA ($\sim$ 12 µM) was mixed with the Exo-Fect™ reagent according to manufacturer's instructions. Events were recorded for 1 min on three different channels 488/10 (side scatter), 525/40 and 670/30 (bandpass filters) and collected with a single-photon counting avalanche photodiode detector (SPCM APD).

For FAM-siRNA and Atto 488 DOPE experiments the blue 488 nm laser was set 10/50 mW and the red 638 nm laser was set 40/100 mW with a SS decay of 0.2 %. For Cy5-FLuc-mRNA experiments, the 488 nm laser was changed to 20/50 mW and the 638 nm laser to 20/100 mW. Most samples were diluted 1 to 50 (v/v) with PBS before measurement except for the Exo-Fect™ transfected samples which were diluted 1 to 80 (v/v) with PBS. The samples were processed using the NanoFCM™ Professional Suite software (version 1.8, NanoFCM Co., Ltd, UK) and the data was analyzed with the FlowJo™ v10 software (BD Biosciences, USA)

## GFP knock down experiments

For GFP knock down experiments, 30,000 HeLa-GFP cells (Cell Biolabs, Inc., USA) were seeded per well inside a 24-well plate. After 24 h of incubation at 37 °C and 5 % $CO_2$ the cells were treated with LNVs, or ssEVs that were loaded with either the GFP-siRNA (siGFP) or a scrambled, control siRNA (siCTRL). Lipofectamine™ RNAiMAX (Thermo Fisher Scientific, USA) with GFP-siRNA and naked GFP-siRNA served as a positive and negative control, respectively. All the samples were prepared in 100 μL OptiMEM™ and added to 400 μL of 10 % (v/v) serum-containing DMEM to reach a final siRNA concentration of 10 nM for each condition. After additional 48 h of incubation the GFP expression was analyzed using a CytoFLEX™ flow cytometry instrument (Beckman Coulter, USA). Cells were then washed twice with PBS and trypsinized. Cells were collected in serum-containing DMEM and centrifuged at 300 g for 5 min. The cell pellets were washed once with PBS and treated with a Zombie NIR™ Fixable Viability Kit (Biolegend, USA) according to manufacturer's instructions. After 15 min of incubation, the cells were pelleted by centrifugation at 300 g for 10 min and resuspended in 120 μL of ice-cold flow cytometry buffer (PBS with 0.5 % (w/v) BSA and 2 mM EDTA) for analysis. Data were processed using the FlowJo™ v10 software.

Luciferase expression

For luciferase experiments, 5,000 HeLa cells were seeded per well in a 96-well plate one day prior to transfection. After 24 h of incubation at 37 °C and 5 % $CO_2$ the culture medium was replaced with 90 μL of FBS. The cells were then treated with different concentrations of FLuc-mRNA-loaded LNVs and ssEVs. Lipofectamine™ MessengerMAX™ (Thermo Fisher Scientific, USA) with FLuc-mRNA and naked FLuc-mRNA served as positive and negative controls, respectively. For each condition, 12 uL of sample in PBS was added to the wells, so that a final mRNA dose of 100, 10, 1 and 0.1 ng per well was added to the cells and the final serum concentration was ~ 90 % (v/v). After 24 h of incubation, the cells were washed twice with DPBS (Dulbecco's phosphate-buffered saline) before the cells were lysed and the luciferase expression was assayed using the Pierce™ Firefly Luciferase Glow Assay Kit (Thermo Fisher Scientific, USA). The luminescence measurements were carried out with a Tecan Spark® microplate reader and normalized to the loaded protein amount as determined by the Micro BCA™ Protein Assay Kit (Thermo Fisher Scientific, USA).

EXAMPLE 2: Impact of lipid ratio, pH and N/P on physicochemical characterization of unloaded vs siRNA-loaded LNVs

Unloaded LNVs and siRNA-loaded LNVs were prepared using various molar concentrations of MC3 (40 %, 50 % and 60 %) at pHs 5 and 7.4 as described in Example 1.

The hydrodynamic diameter ($d_h$) of the unloaded LNVs and siRNA-loaded LNVs increased in LNVs containing a higher molar concentration of MC3 or prepared with a more alkaline pH (FIG. 1B (unloaded LNVs), FIG. 1D (siRNA-loaded LNVs)). The ζ-pot decreased in unloaded LNVs, and siRNA loaded LNVs prepared with a more alkaline pH (FIG. 1F). The polydispersity index (PDI) did not significantly vary with MC3 percentage or pH in the unloaded LNVs (FIG. 1C (unloaded LNVs)) or the siRNA loaded LNVs (FIG. 1E (siRNA-loaded LNVs)). The different N/P ratios did not significantly affect the size and PDI (FIGs. 1M-N) in siRNA loaded LNVs. The presence of siRNA entrapped into LNVs was confirmed with EMSA in the presence or absence of Triton™ X-100 (FIG. 1O). siRNA was detected only when LNVs were solubilized with Triton™ X-100.

The loading efficiency (LE %) of the siRNA was not affected by the tested varying N/P ratios and MC3 molar concentrations (FIGs. 1P-Q). The integrity of siRNA-loaded in LNVs (N/P 25, 60% MC3 and pH 7.4) was confirmed (FIG. 1R)

Cryogenic transmission electron microscopy (cryo-TEM) images show LNVs and siRNA-loaded LNVs having a size of ~ 100 nm and a non-lamellar structure, both at pH 7.4 and 5 (FIGs. 1G-J). Small-angle X-ray scattering (SAXS) of LNVs and siRNA-loaded LNVs (N/P 25, 60 mol % MC3) revealed more detailed information on the particle structure (FIGs. 1K-L). At pH 7.4, both LNVs and siRNA-loaded LNVs show a sponge-like structure based on L3 phases. On the other hand, at pH 5, LNVs formed hexosomes based on inverted hexagonal phases (HII) (FIG. 1K). Once siRNA was added, the structure based on HII phases became more disordered and a fourth peak appears (FIG. 1K, dark line).

EXAMPLE 3: Physicochemical characterization of ssEVs produced with siRNA-loaded LNVs

In two different experimental settings, CellTrace™ Far Red (CT)-labeled EVs were mixed with either FAM-siRNA-loaded LNVs (FIG. 2G) or Atto 488 DOPE-labeled LNVs (FIG. 2I) using LNVs (60% MC3; N/P 25) at 37 °C and 4 °C, pH 7.4 for 30 min, in accordance with the method described in Example 1. (FIGs. 2G-I) The double positive population (in the square insert) appears upon mixing LNVs and EVs and confirm the formation of ssEVs. This population was not present when, for instance, only naked FAM-siRNA was added to EVs (FIG. 2G) or when the Exo-Fect™ Exosome Transfection Kit (FIG. 2H) was used or in all other tested control samples. (FIG. 2J) shows that the loading of FAM-siRNA into ssEVs by using LNVs is significantly more efficient than that achieved with the commercial Exo-Fect™ Kit. (FIGs. 2I) shows that the double positive population appears also (in the square inset) when Atto 488 DOPE-labeled LNVs are mixed with CellTrace™-labeled EVs, confirming the ssEV formation. (FIG. 2K) illustrates the percentage of the double positive ssEV population over the total particle population, hence the transfer of Atto 488 DOPE from LNVs to EVs, confirming the ssEV formation.

LNVs spontaneously and instantaneously produced a fusion stalk in EVs during the hybridization step at pH 7.4 when the LNVs exist as sponges (FIGs. 1H and 1L), generating hybrid systems referred to herein as semi-synthetic extracellular vesicles (ssEVs) as shown in FIGs. 2A-E (number ratio siRNA-loaded LNVs to EVs of 4:1, LNVs having been prepared with a N/P ratio of 25 and 60 mol% MC3). The interaction was also confirmed by nanoparticle flow cytometry (NanoFCM) measurements (FIGs. 2G-I). By contrast, a massive aggregation between LNVs and EVs occurred at pH 5 (FIG. 2F) which did not result in ssEV formation.

The impact of LNV:EV number ratios, reaction temperature (4°C, 25°C, and 37°C), and pH (5, 6, 7.4 and 9) on generated particle sizes and number was determined with a DLS/SLS plate reader in 384-microwell plates. FIG 2L shows that the different number ratios do not affect the ssEV size population and reaction kinetics as determined by an increase in the hydrodynamic diameter and the particle concentration over time (1 h). FIG. 2M shows that the velocity of the hybridization process depends on the temperature, being quicker at 37 °C than at 4 °C, whereas FIG. 2N shows that ssEV size increases when the pH is decreased. Further, at pH 5 the particle concentration could not be detected after 5 min, possibly due to presence of multiple particle species including larger aggregates.

EXAMPLE 4: Impact of pH on physicochemical characterization of unloaded vs mRNA-loaded LNVs and physicochemical characterization of ssEVs produced with mRNA loaded LNVs

Unloaded LNVs and mRNA-loaded LNVs were prepared at pH values of 5 and 7.4 as described in Example 1 and used to generate ssEVs.

The produced mRNA-loaded LNVs (N/P 25 and 60 mol % MC3) had a mean hydrodynamic diameter of ~ 130 nm (FIG. 3A), a low polydispersity index (PDI) value of about 0.11 (FIG. 3B). Data also shows that the $\zeta$-pot of the LNVs slightly decreases once mRNA is loaded into the particles, both at acidic and neutral pH (FIG. 3C). mRNA was entrapped into LNVs with a loading efficiency (LE) of 94 % $\pm$ 5 (FIG. 3D).

The mRNA-loaded LNVs' structure is shown in cryo-TEM micrographs (number ratio mRNA-loaded LNVs : EVs of 4:1, LNVs having been prepared with a N/P ratio of 25 and 60 mol% MC3)(FIG. 3E).

The fusion process of mRNA-loaded LNVs with EVs is also shown in cryo-TEM micrographs where it can be seen that similar to siRNA-loaded LNVs, mRNA-loaded LNVs spontaneously fused with EVs forming ssEVs at 37°C and pH 7.4, i.e. under very mild conditions (FIG. 3F). The loading of the mRNA in the ssEVs is confirmed in FIGs. 3G-H. As described above CFSE-labeled EVs were incubated with either Cy5-FLuc-mRNA-loaded LNVs or with FLuc-mRNA-loaded LNVs. A double positive population (in the square inset) appears upon mixing Cy5-mRNA-loaded LNVs and CFSE-labeled EVs and suggesting the formation of mRNA-loaded ssEVs. This population was not present when, for instance, only naked Cy5-FLuc-mRNA was added to EVs or when the unlabeled Fluc-mRNA was encapsulated within LNVs and mixed with unlabeled EVs.

EXAMPLE 5: Impact on viability of HeLa cells treated with LNVs, and siRNA-loaded LNVs in an *in vitro* model

HeLa cells were cultured at 37 °C and their viability was tested with the method described in Example 1.

Both unloaded LNVs and siRNA-loaded LNVs did not lead to a decrease in cell viability at all the tested concentrations and time points (FIGs. 4A-B).

EXAMPLE 6: Retention of the bioactivity of EV membrane proteins after hybridization

The enzymatic activity of EV membrane proteins CD73 and DPP4 was determined as described in Example 1. It was shown not to be altered after hybridization with siRNA-loaded LNVs at 4 °C and 37 °C (FIGs. 5A-B). By contrast, at pH 5, the enzymatic activity of CD73 was significantly reduced after hybridization with siRNA-loaded LNVs (FIG. 5A). The Exo-Fect™ reagent also significantly reduced the enzymatic activity of both enzymes (CD73 and DPP4) after hybridization compared to ssEVs (4 °C and 37 °C at pH 7.4) (FIGs. 5A-B).

EXAMPLE 7: Transfection efficacy of siRNA-loaded ssEVs in GFP-expressing HeLa cells

Untreated cells (that received only the medium), PBS, free GFP-siRNA represent the negative controls and cells treated with Lipofectamine™ RNAiMAX with GFP-siRNA represent the positive control, respectively. Successively, the transfection efficacy of GFP-siRNA-loaded LNVs vs GFP-siRNA-loaded ssEVs was assessed in HeLa-GFP cells in the presence of ~ 8 % (v/v) FBS. The results show that GFP-siRNA-loaded LNVs (LNVs (w siGFP)) reduced the normalized GFP fluorescence intensity by roughly 59 %, and that EVs significantly enhanced the LNVs transfection efficacy after hybridization showing a GFP fluorescence decrease of ~ 75 % (ssEVs (w siGFP)), which is equal to that of the positive control Lipofectamine™ RNAiMAX (RNAiMAX (w siGFP)) (FIG. 6).

EXAMPLE 8: Transfection efficacy of mRNA-loaded ssEVs in HeLa cells

PBS-treated cells and cells that received naked FLuc-mRNA represent the negative controls and cells treated with Lipofectamine™ MessengerMAX™ with FLuc-mRNA represent the positive control, respectively. Transfection efficiencies of different mRNA doses (100, 10, 1 and 0.1 ng) encapsulated in either LNVs, ssEVs or Lipofectamine™ MessengerMAX™ were assessed in HeLa cells in the presence of ~ 90 % (v/v) serum. The results show that ssEVs significantly improved the expression of luciferase compared to LNVs as shown by a luminescence signal increase of around seven-fold for an mRNA dose of 100 ng and around ten-fold for an mRNA dose of 10 ng (FIG. 7).

EXAMPLE 9: Nanostructures prepared with KLP and DOPE

Nanostructures similar to multilamellar liposomes were produced with DOPE and KLP but without MC3, as shown in FIG. 8.

EXAMPLE 10: Nanoparticles prepared with DOPC

Nanoparticles were produced with MC3, DOPC, a phospholipid displaying a CPP ~ 1 and KLP and their $d_h$ and PDI were evaluated with DLS at pH values of 5 and 7.4 (FIGs. 9A and 9B). Cryo-TEM images (FIGs. 9C-E) shows DOPC-based particles (FIG. 9C) and siRNA/DOPC-based particles (FIG. 9D) having a multivesicular structure. Such a result reveals the fundamental role of phospholipids displaying a CPP value > 1 (e.g., DOPE) in forming non-lamellar-based particles. Further, FIG. 9E suggests that siRNA/DOPC-based nanoparticles do not form ssEVs with MSC-derived EVs.

EXAMPLE 11: Nanoparticles prepared with MPPC

Nanoparticles were produced with MC3, DOPE and MPPC, a lysolipid with CPP < 1. The nanoparticles size distribution was evaluated with a DLS instrument at pH 7.4 (FIG. 10). Particles with a broad size distribution comprising more than one particle population were detected, demonstrating the key role of the non-ionic surfactant (e.g., KLP) in forming particles with appropriate size and PDI values.

EXAMPLE 12: Nanoparticles prepared with DOTMA

Nanoparticles were produced with DOTMA, a fixed cationic lipid, DOPE and KLP and their $d_h$ and PDI were evaluated with the DLS at pH 5 and 7.4 (FIGs. 11A and 11B). Cryo-TEM images (FIG. 11C) show that siRNA-loaded DOTMA particles form massive aggregates in the presence of MSC-derived EVs.

The scope of the claims should not be limited by the embodiments set forth in the examples, but should be given the broadest interpretation consistent with the description as a whole.

REFERENCES

Alvarez-Erviti, L., Seow, Y., Yin, H., et al. 2011. Delivery of siRNA to the mouse brain by systemic injection of targeted exosomes. Nat. Biotechnol., 29, pp. 341-345.

Barragan Vidal, I.A., Müller, M., Generalization of the swelling method to measure the intrinsic curvature of lipids, J. Chem. Phys. 147, 224902, 2017.

Hamai, C., Yang, T., Kataoka, S., Cremer, P.S., Musser, S. M., Effect of Average Phospholipid Curvature on Supported Bilayer Formation on Glass by Vesicle Fusion. Biophys. J. 90, 1241, 2006.

Lamichhane, T. N., Jeyaram, A., Patel, D. B., et al., 2016. Oncogene Knockdown via Active Loading of Small RNAs into Extracellular Vesicles by Sonication. Cell. Mol. Bioeng., 9, pp. 315-324.

O'Brien, K., Breyne, K., Ughetto, S., Laurent, L.C., 2020. RNA delivery by extracellular vesicles in mammalian cells and its applications. Nat. Rev., 21, pp. 585-606.

Rand, R. P., Fuller, N. L., Gruner S. M. and Parsegian, V. A., Membrane Curvature, Lipid Segregation, and Structural Transitions for Phospholipids under Dual-Solvent Stress, Biochemistry, 29, 76, 1990.

Sodt, J., Pastor, R. W., 2013, Bending Free Energy from Simulation: Correspondence of Planar and Inverse Hexagonal Lipid Phases, Biophys. J., 104 (10), 2202-11.

Théry et al., 2018, Minimal information for studies of extracellular vesicles, J Extracellular Vesicles, 7(1): 1535750. Wu et al., 2023. "Advances in Extracellular Vesicle Nanotechnology for Precision Theranostics" Adv. Sci. 2023, 10, 2204814.

**Claims**

1. A pH-inducible structure-switching non-lamellar lipid nanovector (LNV) comprising:

   (a) at least one ionizable cationic lipid;
   (b) at least one phospholipid displaying a critical packing parameter (CPP) value > 1; and
   (c) at least one non-ionic surfactant displaying a CPP value < 1, at a molar concentration of between 10 % and 50 %.

2. The LNV of claim 1, wherein:

   (a) the at least one ionizable cationic lipid has an apparent acid dissociation constant (pKa) prior to incorporation in LNV of between 5 and 7.5;
   (b) the at least one phospholipid is a phosphatidylethanolamine (PE)-based lipid;
   (c) the at least one surfactant is a polyethylene glycol ester of fatty acid or a polysorbate; or
   (d) a combination of at least two of (a) to (c).

3. The LNV of claim 1 or 2, wherein:

   (a') the at least one ionizable cationic lipid is D-Lin-MC3-DMA (MC3);
   (b') the at least one phospholipid is 2-dioleoyl-sn-glycero-3-phosphoethanolamine (DOPE);
   (c') the at least one non-ionic surfactant is a polyethylene glycol ester of fatty acid such as polyethylene glycol 12-hydoxystearate, preferably Kolliphor® HS15 (KLP); or
   (d') a combination of at least two of (a') to (c').

4. The LNV of any one of claims 1 to 3, loaded with a drug or a diagnostic agent, preferably wherein the drug or diagnostic agent is a nucleic acid molecule.

5. Composition comprising the LNV defined in any one of claims 1 to 4, and at least one pharmaceutically acceptable excipient.

6. Method for making the LNV defined in any one of claims 1 to 3, comprising mixing (a) an organic phase comprising the at least one ionizable cationic lipid, the at least one phospholipid and the at least one surfactant, in an organic solvent; and (b) an aqueous medium.

7. The method of claim 6, wherein at least one of (i) the organic phase further comprises a lipophilic drug or diagnostic agent; and (ii) the aqueous phase further comprises a hydrophilic a drug or diagnostic agent, preferably wherein the hydrophilic drug or diagnostic agent is a nucleic acid molecule.

8. The method of claim 7, wherein (ii) the aqueous phase further comprises a hydrophilic drug or diagnostic agent and the aqueous medium has a pH of between about 4 and about 6.

9. The method of any one of claims 6 to 8, wherein the mixing is performed with a microfluidic device.

10. Method of making a semi-synthetic extracellular vesicle (ssEV) comprising mixing the LNV defined in any one of claims 1 to 4 or the composition defined in claim 5, with an extracellular vesicle (EV) at a pH higher than 6 and up to about 10, whereby the ssEV is generated.

11. Semi-synthetic extracellular vesicle (ssEV) prepared by the method defined in claim 10.

12. Semi-synthetic extracellular vesicle (ssEV) comprising essentially all components, partially or completely mixed, of:

(a) the LNV defined in any one of claims 1 to 4; and
(b) an extracellular vesicle (EV).

13. Composition comprising the ssEV defined in claim 11 or 12, and at least one pharmaceutically acceptable excipient.

14. Kit comprising (A) (a) the LNV defined in any one of claims 1 to 4 or the composition defined in claim 5; and (b) (i) a solution for hydrating (a); (ii) extracellular vesicles (EVs) or a composition comprising the EVs and at least one pharmaceutically acceptable excipient; (iii) instructions for using (a) and at least one of (i) and (ii); or (iv) a combination of at least two of (i) to (iii); or (B) (a') the ssEV defined in claim 11 or 12, or the composition defined in claim 13; and (b') (i') a solution for hydrating (a'); (ii') instructions for using (a') and (i'); or (iii') a combination of (i') and (ii').

15. Use of the ssEV defined in claim 11 or 12 or of the composition defined in claim 13, as a medicament or a diagnostic agent.

FIG. 1A

Organic phase
(ethanol)

MC3
+
DOPE
+
KLP

Aqueous phase
(acetate buffer, pH 4)

RNA

High-precision syringe pump

Aqueous phase

Organic phase

100 nm

FIG. 1C

FIG. 1B

FIG. 1E

FIG. 1D

FIG. 1F

FIG. 1H

FIG. 1G

FIG. 1I

FIG. 1J

LNVs

siRNA/LNVs

100 nm

100 nm

EP 4 516 295 A1

FIG. 1K

FIG. 1L

FIG. 1N

FIG. 1M

FIG. 10

siRNA-loaded LNVs $\Delta$ N/P

| Free siRNA | 50 | 25 | 12 | 6 | Free siRNA |

+ 1 % (v/v) Triton

EP 4 516 295 A1

FIG. 1P

FIG. 1Q

EP 4 516 295 A1

FIG. 1R

EP 4 516 295 A1

FIG. 2A

FIG. 2B

FIG. 2C

FIG. 2D

FIG. 2E

FIG. 2F

FIG. 2G

EP 4 516 295 A1

FIG. 2H

FIG. 2I

FIG. 2K

CellTrace™ Far Red + Atto 488 DOPE + events (%)

ssEVs (37 °C)

FIG. 2J

CellTrace™ Far Red + FAM-siRNA + events (%)

ssEVs (4 °C)
ssEVs (37 °C)
Exo-Fect™

FIG. 2M

FIG. 2L

FIG. 2N

FIG. 3A

FIG. 3B

FIG. 3C

FIG. 3D

FIG. 3E

FIG. 3F

FIG. 3G

| CFSE-EVs | LNVs (w Cy5-mRNA) | CFSE-EVs + free Cy5-mRNA | CFSE-EVs + LNVs (w Cy5-mRNA) | EVs + LNVs (w mRNA) |

FIG. 3H

EP 4 516 295 A1

FIG. 4A

FIG. 4B

FIG. 5A

FIG. 5B

FIG. 6

FIG. 7

mRNA/well
▨ 0.1 ng
▨ 1 ng
■ 10 ng
■ 100 ng

w FLuc-mRNA

FIG. 8

500 nm

FIG. 9A

FIG. 9B

FIG. 9C

FIG. 9D

FIG. 9E

FIG. 10

FIG. 11A

FIG. 11B

FIG. 11C

Europäisches
Patentamt

European
Patent Office

Office européen
des brevets

# EUROPEAN SEARCH REPORT

Application Number

**EP 23 19 5151**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2018/232357 A1 (MODERNATX INC [US]) 20 December 2018 (2018-12-20) | 1-9 | INV. A61K9/51 |
| Y | * the whole document * * claims 1-83 * | 10-15 | |
| X | WO 2020/061457 A1 (MODERNATX INC [US]) 26 March 2020 (2020-03-26) | 1-9 | |
| Y | * the whole document * * claims 1-153; figures 1-8 * | 10-15 | |
| X | WO 2014/172045 A1 (UNIV BRITISH COLUMBIA [CA]) 23 October 2014 (2014-10-23) | 1-9 | |
| Y | * the whole document * * claims 1-46; figures 1-12 * | 10-15 | |
| Y | WO 2022/069577 A1 (EVOX THERAPEUTICS LTD [GB]) 7 April 2022 (2022-04-07) * the whole document * * claims 1-30 * | 1-15 | |
| Y | JOSEPHA ROERIG ET AL: "Standardization Approaches for Extracellular Vesicle Loading with Oligonucleotides and Biologics", SMALL, WILEY, HOBOKEN, USA, vol. 19, no. 40, 7 June 2023 (2023-06-07), page n/a, XP072511490, ISSN: 1613-6810, DOI: 10.1002/SMLL.202301763 * the whole document * | 1-15 | TECHNICAL FIELDS SEARCHED (IPC) A61K |

The present search report has been drawn up for all claims

| Place of search | Date of completion of the search | Examiner |
|---|---|---|
| Munich | 30 January 2024 | Felder, Christian |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
........................................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.                EP 23 19 5151

This annex lists the patent family members relating to the patent documents cited in the above-mentioned European search report.
The members are as contained in the European Patent Office EDP file on
The European Patent Office is in no way liable for these particulars which are merely given for the purpose of information.

30-01-2024

| Patent document cited in search report | | | Publication date | Patent family member(s) | | Publication date |
|---|---|---|---|---|---|---|
| WO 2018232357 | A1 | | 20-12-2018 | EP | 3638215 A1 | 22-04-2020 |
| | | | | MA | 49421 A | 22-04-2020 |
| | | | | US | 2021145982 A1 | 20-05-2021 |
| | | | | US | 2023330260 A1 | 19-10-2023 |
| | | | | WO | 2018232357 A1 | 20-12-2018 |
| WO 2020061457 | A1 | | 26-03-2020 | CA | 3113651 A1 | 26-03-2020 |
| | | | | CN | 113271926 A | 17-08-2021 |
| | | | | EP | 3852728 A1 | 28-07-2021 |
| | | | | JP | 2022501367 A | 06-01-2022 |
| | | | | US | 2021378980 A1 | 09-12-2021 |
| | | | | WO | 2020061457 A1 | 26-03-2020 |
| WO 2014172045 | A1 | | 23-10-2014 | CA | 2906732 A1 | 23-10-2014 |
| | | | | CN | 105143456 A | 09-12-2015 |
| | | | | EP | 2971013 A1 | 20-01-2016 |
| | | | | JP | 6605446 B2 | 13-11-2019 |
| | | | | JP | 2016515815 A | 02-06-2016 |
| | | | | US | 2016022580 A1 | 28-01-2016 |
| | | | | US | 2019307689 A1 | 10-10-2019 |
| | | | | WO | 2014172045 A1 | 23-10-2014 |
| WO 2022069577 | A1 | | 07-04-2022 | CA | 3188534 A1 | 07-04-2022 |
| | | | | CN | 116348148 A | 27-06-2023 |
| | | | | EP | 4222273 A1 | 09-08-2023 |
| | | | | JP | 2023545658 A | 31-10-2023 |
| | | | | KR | 20230078723 A | 02-06-2023 |
| | | | | US | 2023355805 A1 | 09-11-2023 |
| | | | | WO | 2022069577 A1 | 07-04-2022 |

EPO FORM P0459

For more details about this annex : see Official Journal of the European Patent Office, No. 12/82

# EP 4 516 295 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2017151333 A1

### Non-patent literature cited in the description

- **ALVAREZ-ERVITI, L** ; **SEOW, Y** ; **YIN, H et al.** Delivery of siRNA to the mouse brain by systemic injection of targeted exosomes. *Nat. Biotechnol.*, 2011, vol. 29, 341-345
- **BARRAGAN VIDAL, I.A.** ; **MÜLLER, M**. Generalization of the swelling method to measure the intrinsic curvature of lipids. *J. Chem. Phys*, 2017, vol. 147, 224902
- **HAMAI, C.** ; **YANG, T** ; **KATAOKA, S** ; **CREMER, P.S** ; **MUSSER, S. M**. Effect of Average Phospholipid Curvature on Supported Bilayer Formation on Glass by Vesicle Fusion. *Biophys. J*, vol. 90 (1241), 2006
- **LAMICHHANE, T. N.** ; **JEYARAM, A.** ; **PATEL, D. B. et al.** Oncogene Knockdown via Active Loading of Small RNAs into Extracellular Vesicles by Sonication. *Cell. Mol. Bioeng*, 2016, vol. 9, 315-324
- **O'BRIEN, K** ; **BREYNE, K** ; **UGHETTO, S.** ; **LAURENT, L.C.** RNA delivery by extracellular vesicles in mammalian cells and its applications. *Nat. Rev.*, 2020, vol. 21, 585-606
- **RAND, R. P** ; **FULLER, N. L** ; **GRUNER S. M** ; **PARSEGIAN, V. A**. Membrane Curvature, Lipid Segregation, and Structural Transitions for Phospholipids under Dual-Solvent Stress. *Biochemistry*, 1990, vol. 29, 76
- **SODT, J** ; **PASTOR, R. W**. Bending Free Energy from Simulation: Correspondence of Planar and Inverse Hexagonal Lipid Phases. *Biophys. J.*, 2013, vol. 104 (10), 2202-11
- **THÉRY et al.** Minimal information for studies of extracellular vesicles. *J Extracellular Vesicles*, 2018, vol. 7 (1), 1535750
- **WU et al.** Advances in Extracellular Vesicle Nanotechnology for Precision Theranostics. *Adv. Sci.*, 2023, vol. 10, 2204814